(19) Europäisches Patentamt / European Patent Office / Office européen des brevets

(11) **EP 4 239 648 B1**

(12) **EUROPÄISCHE PATENTSCHRIFT**

(45) Veröffentlichungstag und Bekanntmachung des Hinweises auf die Patenterteilung:
**29.01.2025  Patentblatt 2025/05**

(21) Anmeldenummer: **23182789.0**

(22) Anmeldetag: **10.09.2019**

(51) Internationale Patentklassifikation (IPC):
*G16H 50/20* *(2018.01)*

(52) Gemeinsame Patentklassifikation (CPC):
**G16H 50/20**

(54) **VERFAHREN ZUR HERZÜBERWACHUNG**

METHOD FOR CARDIAC MONITORING

PROCÉDÉ DE SURVEILLANCE CARDIAQUE

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität:  **10.09.2018  DE 102018121974**

(43) Veröffentlichungstag der Anmeldung:
**06.09.2023  Patentblatt 2023/36**

(62) Dokumentnummer(n) der früheren Anmeldung(en) nach Art. 76 EPÜ:
**19789857.0 / 3 850 640**

(73) Patentinhaber: **Cardisio GmbH**
**60549 Frankfurt (DE)**

(72) Erfinder:
• **Baumeister, Meik**
**79206 Breisach (DE)**
• **Tenderich, Gero**
**47918 Tönisvorst (DE)**

(74) Vertreter: **Dammertz, Ulrich**
**Patentanwaltskanzlei Dammertz**
**Ferdinand Strasse 10**
**47906 Kempen (DE)**

(56) Entgegenhaltungen:
EP-A2- 0 086 429        WO-A1-03/057031
CN-A- 108 420 454        DE-A1- 102012 106 893
JP-A- 2008 220 556

• BLOOMFIELD D K ET AL: "R and T wave relationships in the lead I electrocardiogram", JOURNAL OF ELECTROCARDIOLOGY, ELSEVIER SCIENCE, XX, vol. 2, no. 2, 1 April 1969 (1969-04-01), pages 167 - 170, XP026701536, ISSN: 0022-0736, [retrieved on 19690401], DOI: 10.1016/S0022-0736(69)81021-6

**Beschreibung**

Die Erfindung betrifft ein Herzüberwachungsverfahren nach dem Oberbegriff von Anspruch 1.

Zu den häufigsten Erkrankungen von Patienten zählt die Koronare Herzkrankheit, kurz KHK. Die derzeit hierzu vorliegende Prävalenz in Deutschland (bei Männern ca. 30%, bei Frauen ca. 15%) belegt einen großen Bedarf an Geräten, mit denen solche Probleme schnell erkannt und die Menschen bzw. Patienten einer frühen Therapie oder Beratung zugeführt werden können. Ein weiteres großes Feld der Störungen in der Arbeit des Herzens betrifft die Arrhythmie oder Herzrhythmusstörungen. Das Herz eines Menschen besitzt ein kompliziertes Erregungsbildungs- und Leitsystem (Erregungssystem), welches der Treiber der Herzbewegungen ist. Durch die Teilung des Herzens in die Kammern und Vorhöfe ist es notwendig, den Blutfluss durch Muskelkontraktionen zu regulieren. Im Zusammenhang mit der rhythmischen Kontraktion des Herzmuskels besteht das Erregungssystem aus speziellen Muskelzellen, welche in der Lage sind, sich spontan selbst zu de- und repolarisieren. Damit kann ein Rhythmus, der in erster Linie vom Sinusknoten generiert wird, aufrechterhalten werden. Bei einer Störung dieses Erregungssystem kommt es zu Beeinträchtigungen der Pumpleistung des Herzens und somit zu Versorgungsengpässen. Nicht alle Symptome sind lebensgefährlich, können aber, sobald sie häufig auftreten, gute Indikatoren für drohende lebensgefährliche Zustände werden.

Der Referenzstandard zur Erkennung einer KHK ist die Koronarangiographie, allerdings wird hiermit der Bereich der Früherkennung der KHK verlassen. Hierbei wird ein Katheter in das Herz eingeführt, um u.a. eine Druckmessung der Aorta durchzuführen. Weiterhin werden Kontrastmittel verwendet, die die Darstellung der Koronararterien des Herzens erlauben. Entsprechend handelt es sich um eine invasive Methode mit den damit verbundenen gesundheitlichen Risiken. Alternativen dazu sind eine Angiographie mittels CT und MRT. Bei diesen Alternativen werden ebenfalls Kontrastmittel verwendet, mit den damit verbundenen gesundheitlichen Risiken. Diese Verfahren sind nachteilig mit einem hohen Aufwand und großen Kosten verbunden.

Gemessen am goldenen Standard der Koronardiagnostik der Koronarangiographie mit einer Spezifität und Sensitivität von 100 %, zeigen nicht invasive Verfahren wie das Ruhe-EKG und die Ruhe-Echokardiographie eine deutlich geringere Sensitivität und Spezifität (von weniger als 30% bzw. 70%). Erst unter Belastung erhöhen sich diese für beide Verfahren, machen aber jeweils die Anwesenheit eines Arztes notwendig. Ferner sind die Ergebnisse im Fall der Stressechokardiographie zusätzlich Untersucher abhängig. Hinzu kommt, dass beide dieser Verfahren zusätzlich einen Zeitraum von mindestens 15-30 Minuten benötigen. Weitere nicht invasive Verfahren wie Myokard-perfusionssyntigraphie (MPS), Koronarcomputer-Tomographie (CCT) oder kardiale Magnetresonanztomographie (MRT) erfordern einen erheblichen apparativen sowie personellen Aufwand, die Anwesenheit eines Arztes und stellen ferner einen erheblichen Kostenfaktor dar.

Für eine nicht-invasive Untersuchung von Patienten kann eine EKG- Untersuchung durchgeführt werden. Auf diesem Gebiet bieten moderne Softwaresysteme einem Arzt viele Werkzeuge an, um eine Vermessung von Patienten zumindest schnell durchzuführen. Gleichwohl ist die Auswertung eines EKG maßgeblich von der Erfahrung des behandelnden Arztes abhängig und damit unsicher. Weiterhin kann eine Überlastung von Ärzten zu Fehldiagnosen führen. Zur Lösung dieser Problematik haben sich im Bereich der EKG-Analyse automatische Vermessungssysteme etabliert. Allerdings beschränken sich diese automatischen Systeme auf die Vermessung von EKGs und bilden somit die Erfahrung beziehungsweise definierte Parameter (bspw. QT-Zeit oder ST-Hebung) in der Software ab. Es ist somit nur die Unsicherheit durch den Faktor Mensch entfernt worden.

Die Parameter eines EKG, vor allem in Ruhe, sind zur Erkennung von KHK in der Regel unzureichend. Aus diesem Grund wird mit Patienten ein Belastungs-EKG durchgeführt, um damit die Früherkennung einer KHK zu ermöglichen. Dafür ist es erforderlich, dass der Patient einer körperlichen Belastung ausgesetzt wird. Es erfolgt somit eine Provokation der Symptome, die zum Herzinfarkt führen können. Dies macht bei einer solchen Messung stets die Anwesenheit eines Arztes notwendig. Der Nachteil einer solchen Vorgehensweise liegt in den damit verbundenen Risiken. Abgesehen von der Gefahr für den Patienten ist es nicht für alle Menschen möglich solche Tests durchzuführen. Vor allem in den Risikogruppen (Diabetes, Übergewicht, hohes Alter) sind die Menschen nicht immer in der Lage, die notwendige Belastung überhaupt zu erreichen, mangels ausreichender Kondition. Es gibt als Alternative die Belastung durch Medikamente und dann Untersuchung mittels Echokardiographie (Stress-Echo) mittels Ultraschall. Allerdings ist das Echo eher zur Untersuchung von Bewegungsstörungen geeignet und bietet ebenfalls nicht die Sicherheit einer automatischen Auswertung.

Mit derzeit verfügbaren EKG-Geräte ist es nicht möglich, frühzeitig Anzeichen von KHK zu erkennen oder dem Arzt in geeigneter Form darzustellen. Allen bislang bekannten Geräten ist gemein, dass sie mehrere Ableitungen gleichzeitig darstellen, mit zumeist 3 bis 12 Kanälen. Die Software zur Darstellung erlaubt in der Regel die Konfiguration der Ansicht, welche es einem Arzt erlaubt, sich auf einzelne Ableitungen zu konzentrieren und entsprechend bekannter Richtlinien auszuwerten. Der Verdacht auf einen drohenden Herzinfarkt (das Ergebnis einer unbehandelten KHK) entsteht u.a. bei ST-Streckenhebungen >= 0,1 mV in mindestens zwei Extremitätenableitungen und >= 0,2 mV in zwei benachbarten Brustwandableitungen. Allerdings bedeutet das Fehlen dieser Hebung nicht, dass ein Herzinfarkt ausgeschlossen ist, denn der überwiegende Teil der Herzinfarkte sind Nicht-Hebungsinfarkte (NSTEMI).

In dem Artikel "R and T wave relationships in the lead I Electrocardiogram" der Autoren BLOOMFIELD D K et.al. (JOURNAL OF ELECTROCARDIOLOGY, ELSEVIER SCIENCE, XX, Bd. 2, Nr. 2, 1. April 1969 (1969-04-01), Seiten 167-170, XP026701536, ISSN: 0022-0736, DOI: 10.1016/S0022-0736(69)81021-6) werden die Beziehungen zwischen R- und T-Wellen im Elektrokardiogramm der Ableitung I beschrieben. Es wurde eine signifikante Korrelation zwischen R- und T-Wellenamplituden nachgewiesen, wobei das normale R/T-Verhältnis mit 4,0 $\pm$ 1,6 bestimmt wurde. Wenn offensichtliche Diagnosen wie Myokardinfarkt, Rechtsschenkelblock und Perikarditis zu den durch das R/T-Verhältnis erkannten abnormalen Elektrokardiogrammen hinzukommen, verbessert sich die Erkennungsrate durch das Ableitung-I-Screening auf 84 % der Abnormalitäten. Hieraus folgt, dass das Elektrokardiogramm der Ableitung I eine bedeutende Rolle beim Massenscreening hat.

Aus EP 86 429 B1 ist ein Verfahren zur Kardiogoniometrie bekannt, dass zur Klasse der Vektor-kardiometrischen Methoden gehört. Dieses Verfahren erlaubt eine sehr genaue Ermittlung des das elektrische Feld des Herzens abbildenden Summenvektors und stellt ihn in einem kartesischen Koordinatensystem dar, das an der Herzlängsachse (anstatt an der Körperachse) ausgerichtet ist. Hierbei werden für die Diagnose von Herzkrankheiten die Potentiale, die während einer Depolarisierung und Repolarisierung (R- und T-Schleife) des Herzmuskels maximal erreicht werden, verwendet sowie die Richtungen der entsprechenden zwei potentialmässig ausgezeichneten Vektoren und der Raumwinkel zwischen den beiden ausgezeichneten Vektoren. Dies bedeutet, dass für diagnostische Zwecke bezüglich des Betrags ausgezeichnete Vektoren und getrennt davon deren Richtungen und einzelne Komponenten in kartesischen Koordinaten, Kugelkoordinaten und Zylinderkoordinaten, parametrisiert werden.

Die Ableitungspunkte der Kardiogoniometrie gemäß EP 86 429 B1, zwischen denen Potentialdifferenzen gemessen werden, sind:

- E1 entsprechend dem Punkt für die Spannung V4 (gemäß Wilson),
- E2 sagittal zu E1 und entsprechend dem Punkt für die Spannung V8 (gemäß Wilson),
- E3 lotrecht zur Strecke E1-E2, über E1 in einem Abstand von 0,7-mal dem Abstand zwischen E1 und E2,
- E4 waagrecht von E3 nach der rechten Patientenseite in einem Abstand von 0,7-mal dem Abstand zwischen E1 und E2.

Eine Vektoraddition der gemessenen Potentialdifferenzen ergibt einen Raumvektor V bzw. Projektionen des Raumvektors V auf eine x, y und z-Achse, wobei die x-Achse parallel zur Strecke E1-E2, und die y-Achse parallel zur Strecke E1-E4 und wobei die z-Achse senkrecht auf der durch E1, E2 und E4 aufgespannten Ebene (schrägsagittale Ebene) steht.

Die elektrische Herzaktion erzeugt ein sich über die Zeit änderndes elektrisches Feld. Der gemäß EP 86 429 B1 ermittelte Raumvektor V ist eine Näherung an den tatsächlichen räumlichen Summenvektor dieses Feldes. Dabei entspricht die Richtung des Raumvektors der Feldrichtung und die Länge (Betrag oder Potential) des Raumvektors der Feldstärke. Die drei Spannungen X, Y und Z [mV] stellen die Teilvektoren oder Komponenten des Raumvektors V in einem dreidimensionalen kartesischen Koordinatensystem dar, vorzugsweise den nach der kardiogoniometrischen Methode ermittelten Raumvektor im oben genannten Koordinatensystem. Das Potential berechnet sich als Quadratwurzel aus $(X^2+Y^2+Z^2)$.

Aus WO 99/36860 ist bekannt, dass der zeitliche Verlauf des Betrags des Raumvektors V durch Bestimmung von Maxima und Minima (Nullstellen im Verlauf des Differentialquotienten) in die Bereiche R+, R-, ST, T+ und T- aufgeteilt wird. In diesen Bereichen werden die periodisch registrierten Vektorbeträge aufsummiert und die solcher Art ermittelten Integralwerte für diagnostische Zwecke weiterverwendet. Somit werden schlingenweise Summen von Vektorbeträgen parametrisiert.

Des Weiteren ist aus WO 03/057031 A1 ein Verfahren zur Erzeugung von kardiometrischen Parametern bekannt, die insbesondere für Diagnosezwecke bei der Untersuchung des menschlichen Herzens verwendet werden können. Ein solches Verfahren ist in der WO 03/057031 A1 als "Vektor-Kardiometrie" bezeichnet und dient zur Erzeugung von kardiometrischen Parametern aus einem Vektor, der das elektrische Feld des Herzens abbildet und der z.B. nach bekannten Vektor-kardiometrischen Methoden ermittelt wird. Der Raumvektor wird von einem Koordinatenursprung ausgehend dargestellt, wobei eine von der Vektorspitze beschriebene räumliche Bahn und/oder eine räumliche Geschwindigkeit der sich entlang der Bahn bewegenden Vektorspitze im virtuellen Raum um den Koordinatenursprung parametrisiert wird. Die hierbei erzeugten Parameter bzw. die genannten Abweichungen sind insbesondere für die Diagnose von Herzkrankheiten oder -störungen geeignet.

Die vorstehend erläuterten Verfahren zur Ermittlung und Darstellung eines auf das Herz bezogenen Raumvektors nach der Kardiogoniometrie insbesondere gemäß EP 86 429 B1 unterliegen dem Nachteil, dass Elektroden bzw. Sensoren am menschlichen Körper in exakter Übereinstimmung zu den vier Ableitungspunkten E1-E4, an denen Potentialdifferenzen gemessen werden, anzubringen sind. Eine nicht korrekte Position einer Elektrode insbesondere am Ableitungspunkt E1 führt zu einer Verfälschung der Messwerte, so dass die hieraus gewonnene Analyse unbrauchbar und eine zuverlässige Diagnose für ein untersuchtes Herz hiermit nicht möglich ist.

Ein weiterer Nachteil bei den Technologien der vorstehend genannten Druckschriften zum Stand der Technik besteht darin, dass eine automatische Analyse in Bezug auf die von einem Patienten gewonnenen dreidimensionalen Signale hierbei nicht vorgesehen ist.

Entsprechend liegt der Erfindung die Aufgabe zugrunde, eine Technologie auf dem Gebiet der nicht-invasiven Überwachung des Herzens zu schaffen, mit der in kürzerer Zeit eine verbesserte Untersuchung von Patienten möglich ist und insbesondere auch automatische Analysen möglich sind.

Die obige Aufgabe wird durch ein Verfahren mit den in Anspruch 1 angegebenen Merkmalen gelöst. Vorteilhafte Weiterbildungen der Erfindung sind in den abhängigen Ansprüchen definiert.

Zur Überwachung des Herzens bei einem Menschen ist gemäß der vorliegenden Erfindung ein Verfahren vorgesehen, bei dem auf Grundlage von am Herzen eines Patienten aufgenommenen EKG-Signalen ein Raumvektor anhand der Vektorkardiographie bestimmt wird, wobei dieser Raumvektor den zeitlichen Verlauf des Summenvektors des elektrischen Feldes des Herzens abbildet und eine der Feldrichtung entsprechende Richtung und eine dem Potential entsprechende Länge aufweist. Es wird ein Quotient aus den Flächen, die von einer Länge des Raumvektors (= Radiusvektor) als Funktion der Zeit jeweils während der R-Welle und während der T-Welle überstrichen werden, gebildet, wobei anschließend dieser dimensionslose und skalare Quotient einer weiteren Auswertung zugeführt wird. Im Zuge dieser weiteren Auswertung wird der Quotient gebildet aus der Fläche (R-Welle)/Fläche (T-Welle) jeweils in Bezug zu vorbestimmten Grenzwerten gesetzt, die eine Relevanz für das zu untersuchende Krankheitsbild aufweisen. Dies bedeutet, dass der besagte Quotient gebildet aus der Fläche (R-Welle)/Fläche (T-Welle) mit zumindest einem vorbestimmten Grenzwert korreliert bzw. verglichen wird, auf Grundlage dessen dann eine Diagnose für den untersuchten Patienten in Hinblick auf ein bestimmtes Krankheitsbild (z.B. KHK und/oder HRS und/oder Herzklappeninsuffizienz eines Patienten) erstellt wird bzw. möglich ist. Bei diesem Verfahren wird für das untersuchte Herz eine koronare Herzkrankheit (KHK) erkannt bzw. festgestellt, falls der aus den von dem Raumvektor jeweils während der R-Welle und während der T-Welle überstrichenen Flächen gebildete Quotient

$$\frac{\text{Fläche (R-Welle)}}{\text{Fläche (T-Welle)}} = a$$

nicht im Intervall $[a_{0,KHK}, a_{1,KHK}]$ bzw. ausserhalb dieses Intervalls liegt. Dieses Intervall wird durch einen unteren Grenzwert $a_{0,KHK}$ und durch einen oberen Grenzwert $a_{1,KHK}$ definiert. Diese Grenzwerte $a_{0,KHK}$ und $a_{1,KHK}$ werden in Abhängigkeit von einem Trainingsset bestimmt. Die unteren und oberen Grenzwerte $a_0$ und $a_1$ werden jeweils mit Hilfe eines Trainingssets von Probanden-Daten bestimmt, mit den Schritten:

(i) Berechnung des Quotienten Fläche (R-Welle) / Fläche (T-Welle) für alle Probanden, um damit Ratio-Zeitreihen zu erhalten,
(ii) Berechnung eines Mittelwertes ($\mu$) und einer zugehörigen Standardabweichung ($\sigma$) aus den Ratio-Zeitreihen von Schritt (i),
(iii) Bestimmung des unteren Grenzwertes $a_0$ unter Berücksichtigung des Mittelwertes ($\mu$) und der Standardabweichung ($\sigma$) von Schritt (ii), durch die Beziehung:

$a_0$ = Mittelwert ($\mu$) - Standardabweichung ($\sigma$),
und/oder
Bestimmung des oberen Grenzwertes $a_1$ unter Berücksichtigung des Mittelwertes ($\mu$) und der Standardabweichung ($\sigma$) von Schritt (ii), durch die Beziehung:
$a_1$ = Mittelwert ($\mu$) + Standardabweichung ($\sigma$).

wobei in Schritt (ii) der Mittelwert und die zugehörige Standardabweichung nur für Ratio-Zeitreihen von gesunden Patienten ("- -") berechnet werden.

Des Weiteren wird darauf hingewiesen, dass die Indizierung "KHK" des unteren und oberen Grenzwerts $a_{0,KHK}$ und $a_{1,KHK}$ für die vorliegende Erfindung dahingehend zu verstehen ist, dass mit Hilfe dieser Grenzwerte das Krankheitsbild einer koronaren Herzkrankheit ("KHK") erkannt werden kann, die auch unter der Bezeichnung ischämische Herzkrankheit ("IHK") bekannt ist.

Bei dem soeben diskutierten Verfahren nach der vorliegenden Erfindung kann für das untersuchte Herz auch eine Herzrhythmusstörung (HRS) erkannt werden, falls der aus den von dem Raumvektor (10) jeweils während der R-Welle und während der T-Welle überstrichenen Flächen gebildete Quotient die Bedingung

$$\frac{\text{Fläche (R-Welle)}}{\text{Fläche (T-Welle)}} < a_{0,\,HRS}$$

oder

$$\frac{\text{Fläche (R-Welle)}}{\text{Fläche (T-Welle)}} > a_{1,HRS}$$

erfüllt.

Entsprechend ist es nach dem zuletzt diskutierten Verfahren gemäß der vorliegenden Erfindung möglich, durch einen Vergleich des Quotienten gebildet aus den Flächen, die von einer Länge des Raumvektors (= Radiusvektor) als Funktion der Zeit jeweils während der R-Welle und während der T-Welle überstrichen werden, mit vorbestimmten Werten für den Parameter "a" eine zutreffende Erstdiagnose für das untersuchte Herz eines Patienten zu erstellen, im Hinblick auf ein mögliches Vorliegen einer HRS.

In Abhängigkeit davon, ob konkret das Vorliegen einer HRS oder oder KHK festgestellt werden soll, versteht sich, dass die Grenzwerte $a_0$ und $a_1$ zum Erkennen dieser Krankheitsbilder und damit für einen Vergleich bzw. eine Korrelation mit dem Quotienten gebildet aus der Fläche (R-Welle)/Fläche (T-Welle), der auf entsprechenden Daten basiert, geeignet sind. Im Zusammenhang mit einer HRS kann dies durch die Indizierung "HRS" ausgedrückt werden (d.h. durch $a_{0,HRS}$, $a_{1,HRS}$); im Zusammenhang mit einer KHK kann dies durch die Indizierung "KHK" ausgedrückt werden (d.h. durch $a_{0,KHK}$, $a_{1,KHK}$).

Falls in Bezug auf ein zu untersuchendes Krankheitsbild (z.B. HRS und/oder KHK) belastbare Daten für die unteren und oberen Grenzwerte $a_0$, $a_1$ vorliegen, können diese Grenzwerte vor der Untersuchung eines Patienten auf diese vorbestimmten Werte festgelegt werden, jeweils in Abhängigkeit davon, ob das Vorliegen einer HRS oder einer KHK untersucht werden soll. Alternativ hierzu ist es möglich, diese Grenzwerte mit Hilfe von einem Trainingsset von Datensätzen einer Vielzahl von Probanden zu verwenden, wie nachstehend im Detail erläutert. In Bezug auf die vorstehend genannten Schritte (i) bis (iii) versteht sich, dass diese einem Training zuzuordnen sind, mit dem die unteren und oberen Grenzwerte $a_0$ und $a_1$ mit größerer "Belastbarkeit" bestimmt werden können, um anschließend und auf Grundlage dessen eine tatsächliche Diagnose eines Patienten für das untersuchte Krankheitsbild mit größerer Genauigkeit zu erstellen.

Zur Klarstellung der vorstehend genannten Rechenvorschrift in Schritt (iii) sei nochmals darauf hingewiesen, dass der untere Grenzwert $a_0$ dadurch gebildet wird, dass der Mittelwert $\mu$ und die zugehörige Standardabweichung $\sigma$ voneinander subtrahiert werden. Demgegenüber wird der obere Grenzwert $a_1$ dadurch gebildet, dass der Mittelwert $\mu$ und die zugehörige Standardabweichung $\sigma$ miteinander addiert werden.

In Bezug auf die vorstehend genannte Bestimmung der unteren und oberen Grenzwerte $a_0$ und $a_1$ versteht sich, dass die zugrunde liegenden Daten der Probanden, mit denen gemäß Schritt (i) die Ratio-Zeitreihen gebildet werden, sich jeweils auf das zu untersuchende Krankheitsbild HRS und/oder KHK beziehen bzw. hierfür relevant sind. Beispielsweise werden zur Untersuchung des Vorliegens einer koronaren Herzkrankheit ("KHK") die Grenzwerte $a_{0,KHK}$ und $a_{1,KHK}$ lediglich anhand solcher Datensätze von Probanden bestimmt, von denen mit sicherer Kenntnis ausgeschlossen werden kann, dass sie an einer KHK bzw. IHK erkrankt sind. Mutatis mutandis gilt dies auch für die Grenzwerte $a_{0,HRS}$, $a_{1,HRS}$ bei der Untersuchung eines Patienten hinsichtlich des Vorliegens einer HRS.

Dadurch, dass zur Bestimmung des unteren und oberen Grenzwerts lediglich die Datensätze von gesunden Patienten ("- -") verwendet werden, wird im Vergleich zu den Datensätzen von kranken Patientender Vorteil erreicht, dass die Standardabweichungen der Mittelwerte von den gesunden Patienten in der Regel geringer sind, wodurch das Erkennen der Krankheitsbilder bzw. Befunde HRS und/oder KHK mit größerer Genauigkeit bzw. Zuverlässigkeit möglich ist.

In vorteilhafter Weiterbildung der Erfindung, insbesondere in Bezug auf alle der vorstehend genannten Verfahren, kann vorgesehen sein, dass zur Aufnahme der EKG-Signale ein T-Shirt verwendet wird, welches vier Sensoren bzw. Elektroden aufweist, die einer korrekten Position der vier Ableitungspunkte am Körper des Patienten zugeordnet sind. Diese Elektroden können in den Stoff des T-Shirts geeignet eingearbeitet sein. In gleicher Weise wie ein T-Shirt kann für die Sensoren bzw. Elektroden, die in Kontakt mit dem Körper des Patienten zu bringen sind, auch eine tragbare bzw. mobile Sensorik vorgesehen sein, z.B. in Form eines Brustgurtes oder dergleichen. Wichtig hierbei ist, dass bei einer solchen tragbaren Sensorik die einzelnen Sensoren bzw. Elektroden zu einem Verbund miteinander verbunden sind und hierdurch auch eine genaue Positionierung an den jeweiligen Ableitungspunkten am Körper des Patienten erreicht wird. Entsprechend erübrigt sich damit ein manuelles Anlegen der einzelnen Sensoren am Körper des Patienten.

Die theoretische Grundlage für das Analyseprinzip nach der vorliegenden Erfindung bildet die sogenannte "Vektorkardiographie", die im Bereich der kardialen Ischämie- Diagnostik als nicht-invasive Methode einen nennenswerten Beitrag leistet.

Im Zusammenhang mit dem Verfahren gemäß der vorliegenden Erfindung ist vorgesehen, dass die Darstellung der elektrophysiologischen Eigenschaften des Herzens im Vergleich zur komplizierten Darstellung des oft verwendeten herkömmlichen EKG mit seinen bis zu 12 Ableitungen dadurch vereinfacht wird, dass die gefilterten EKG-Signale auf Grundlage der Vektorkardiographie zunächst in orthogonalisierte Messgrößen überführt werden. Hierbei werden die kardialen Potentiale in einem orthogonalen System gemessen und vektoriell summiert. Bei einer solchen dreidimensionalen Messung und Ortung des Herzpotentials können eine Vielzahl von neuen Parametern geschaffen bzw. berücksichtigt werden, was u.a. die Erkennung der Myokardischämie erlaubt.

Ein wesentlicher Vorteil der Erfindung besteht darin, dass damit eine automatische Analyse unter Verwendung von dreidimensionalen Signalen durchgeführt wird bzw. möglich ist. Hierdurch ist nicht nur eine profunde Analysegrundlage gewährleistet, sondern auch eine grafische Aufbereitung der elektrophysiologischen Eigenschaften des Herzens möglich. Damit wird im Vergleich zu den bislang eingesetzten EKG- Messungen und den dabei verwendeten bis zu 12 Ableitungen (= 12 Dimension) die ansonsten komplizierte Darstellung stark vereinfacht und auf eine intiutive Weise visualisiert, wodurch in der Analyse und Auswertung mit der vorliegenden Erfindung weniger Fehler auftreten.

Zweckmäßigerweise erfolgt die Messung bzw. die nicht-invasive Aufnahme von EKG-Signalen am Herzen des Patienten in dessen Ruhezustand (d.h. ohne körperliche Belastung) unter Verwendung von vier Oberflächenelektroden. Dies bedeutet, dass die EKG-Signale an insgesamt vier Ableitungspunkten am Körper des Patienten in Nähe des Herzens aufgenommen werden. Für diese Messung können folgende Darstellungsformen vorgesehen sein:

- Analoge orthogonale Projektionen X, Y, Z zur Kontrolle der technischen Qualität der Messung;
- Potential, gemessen in den verschiedenen Zeitabschnitten des Herzzyklus;
- Schlingen in 2D und 3D, zur Ortung des Potentials im Herzen,
- Maximalvektoren der Vorhof- und Ventrikel-Depolarisation und der Ventrikel-Repolarisation.

Ein zentraler Aspekt für die vorliegende Erfindung besteht darin, dass die orthogonalisierten Messgrößen in ein System basierend auf Künstlicher Intelligenz (KI), vorzugsweise in Form eines neuronalen Netzes oder einer Mehrzahl von solchen neuronalen Netzen, eingegeben werden, wobei dieses KI-System (bzw. neuronales Netz) mit bereits bekannten Befunden trainiert wurde und insoweit diese Befunddaten von Vergleichspatienten in dem KI-System gespeichert sind. Durch eine funktionale Abbildung der eingegebenen orthogonalisierten Messgrößen über ein neuronales Netzwerkes auf Werte zwischen -1 und 1 wird eine Diagnose für den untersuchten Patienten erstellt, wobei dieses KI-System mit Hilfe von Befunddaten von Vergleichspatienten trainiert wurde, welches typische Charakteristika der Messgrössen entsprechend deren Relevanz für den Gesundheitszustand gewichtet.

Bezüglich der bekannten Befunddaten von Vergleichspatienten, auf welchen das KI-System bzw. neuronale Netz basiert, wird gesondert hervorgehoben, dass für diese Vergleichspatienten eine eindeutige Kenntnis in Bezug auf deren Gesundheitszustand ("gesund" oder "krank") vorliegt. Diese Information kann z.B. mittels einer Koronarangiografie erhalten worden sein, die eine eindeutige Diagnose für die Vergleichspatienten ("gesund" oder "krank") liefert, oder aus anderen Quellen vorliegen. Auf Grundlage dessen erfolgt eine Klassifizierung und ein Clustering der Befunddaten (Diagnose als "gesund" oder "krank").

In vorteilhafter Weiterbildung der Erfindung werden die Ableitungen zwischen den jeweiligen vier Ableitungspunkten, an denen die EKG-Signale aufgenommen werden, sowohl in kartesischen Koordinaten als auch in Kugel- und Zylinderkoordinaten betrachtet. Eine solche Transformation in Kugelkoordinaten ermöglicht die Bestimmung der tatsächlichen Zeitpunkte der physiologischen Extrempunkte (d.h. der genaue Punkt des R-Peaks bzw. der T-Welle) während eines Herzschlags.

Bei dem vorstehend genannten Verfahren gemäß der vorliegenden Erfindung handelt es sich um einen nicht-invasiven, reproduzierbaren, schnell durchführbaren und kostengünstigen Diagnose-Ansatz zur Erkennung hämodynamisch relevanter Stenosen der Koronararterien in Ruhe. Die Diagnose erfolgt über eine computerbasierte infinitesimale, dreidimensionale Verrechnung der Erregungsabläufe des Säugerherzens basierend auf einem spezifischen Algorithmus in Verbindung mit einem KI-System bzw. einem neuronalen Netzwerk in Korrelation zur intrinsischen Blutversorgung sowie der spezifischen räumlichen Ausrichtung des Myokards im Dipolfeld als Funktion der Zeit ausgehend von einem definierten Punkt. Mit einem erfindungsgemäßen Verfahren ist es möglich, in Bezug auf die untersuchten Patienten eine Sensitivität von 95 bis über 99% und eine Spezifität von 80 bis über 90% zu erreichen. Dieser Ansatz wird von der Anmelderin auch als "Cardisiographie" bezeichnet.

In Bezug auf die vorliegende Erfindung wird gesondert hervorgehoben, dass deren Durchführung vorzugsweise unter Verwendung eines Computers oder vergleichbarer Rechnereinheiten erfolgt. Dies gilt für das Verfahren gemäß Anspruch 1 in Bezug auf die Auswertung des Quotienten gebildet aus der Fläche (R-Welle)/Fläche (T-Welle) unter Berücksichtigung von zumindest einem vorbestimmten Grenzwert.

Nachstehend sind verschiedene Ausführungsformen der Erfindung anhand einer schematisch vereinfachten Zeichnung im Detail beschrieben.

Es zeigen:

Fig. 1 — eine vereinfachte Prinzipskizze einer Einrichtung zur Früherkennung des Vorliegens einer KHK oder von HRS,

Fig. 2 — die Ansicht eines Patienten von vorne (Fig. 2a) und von hinten (Fig. 2b),

Fig. 3 — die Ansicht eines T-Shirts von vorne (Fig. 3a) und von hinten (Fig. 3b), das bei der Einrichtung gemäß Fig. 1 verwendbar ist,

Fig. 4, Fig. 7b — jeweils die Ansicht eines Patienten von vorne, zur Verdeutlichung eines orthogonalen Systems (x, y, z), in dem gemäß der vorliegenden Erfindung ein Raumvektor abgebildet wird,

Fig. 5 — schematische vereinfachte Ansichten eines ersten Dreiecks (Fig. 5a) und eines zweiten Dreiecks (Fig. 5b), auf Grundlage derer in Bezug auf das orthogonale System nach Fig. 4 eine Winkelkorrektur durchgeführt wird,

Fig. 6 — eine prinzipiell vereinfachte Ansicht einer System-Architektur, innerhalb derer die Einrichtung von Fig. 1 eingesetzt wird,

Fig. 7a — eine prinzipiell vereinfachte Darstellung eines Raumvektors, der bei einer Aktivität des menschlichen Herzens gebildet wird,

Fig. 8 — ein Ablaufdiagramm, mit dem ein Verfahren, welches nicht Teil der Erfindung ist, durchgeführt wird,

Fig. 9 — ein Ablaufdiagramm, mit dem ein Verfahren, welches nicht Teil der Erfindung ist, gemäß einer weiteren Ausführungsform durchgeführt wird,

Fig. 10 — eine prinzipiell vereinfachte Darstellung einer 3D-Matrix (Fig. 10a) von Messwerten bzw. eine normierte Version hiervon (Fig. 10b), mit den aus den Parameter-Zeitreihen bzw. Parametern abgeleiteten Statistiken, die bei einem Verfahren gemäß Fig. 8 bzw. Fig. 9 zur Anwendung kommt,

Fig. 11a-11d — beispielhafte Zeitreihen-Parameter, die bei einem Verfahren gemäß Fig. 9 anwendbar sind,

Fig. 12 — beispielhafte statistische Verfahren, die bei einem Verfahren gemäß Fig. 9 anwendbar sind,

Fig. 13 — ein Ablaufdiagramm, mit dem ein Verfahren, das nicht Teil der Erfindung ist, durchgeführt wird,

Fig. 14 — ein weiteres Ablaufdiagramm, mit dem ein Verfahren, das nicht Teil der Erfindung ist, gemäß einer weiteren Ausführungsform durchgeführt wird,

Fig. 15 — die Multiplikation einer Berechnungsmatrix mit einem Berechnungsvektor nach einem weiteren Verfahren, das nicht Teil der Erfindung ist,

Fig. 16 — eine prinzipiell vereinfachte Hauptachsentransformation für das Verfahren von Fig. 15,

Fig. 17 — eine vereinfachte Veranschaulichung der Bildung eines Quotienten aus den Flächen, die von einer Länge des Raumvektors (= Radiusvektor) als Funktion der Zeit jeweils während der R-Welle und während der T-Welle überstrichen werden, und

Fig. 18 — eine vereinfachte Veranschaulichung einer Mehrzahl von neuronalen Netzen, die bei einer Anwendung bzw. Durchführung der vorliegenden Erfindung zum Einsatz kommen.

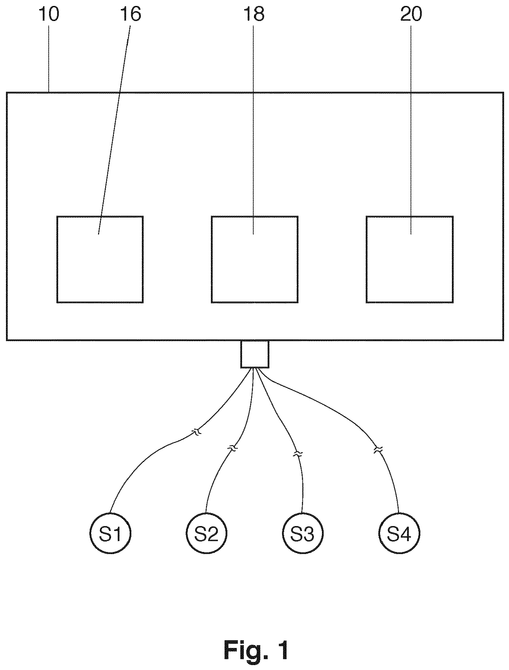

Nachfolgend sind unter Bezugnahme auf die Fig. 1-18 bevorzugte Ausführungsformen von Herzüberwachungsverfahren und einer hierzu eingesetzten Einrichtung 10 im Detail dargestellt und erläutert. Gleiche Merkmale in der Zeichnung sind jeweils mit gleichen Bezugszeichen versehen. An dieser Stelle wird gesondert darauf hingewiesen, dass Darstellungen in der Zeichnung lediglich vereinfacht und insbesondere ohne Maßstab gezeigt sind.

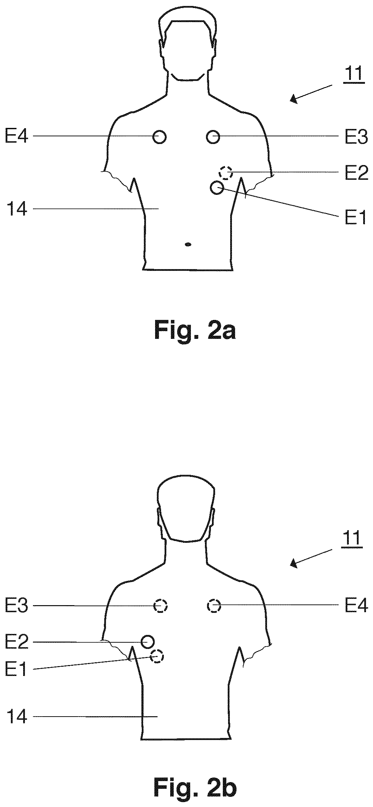

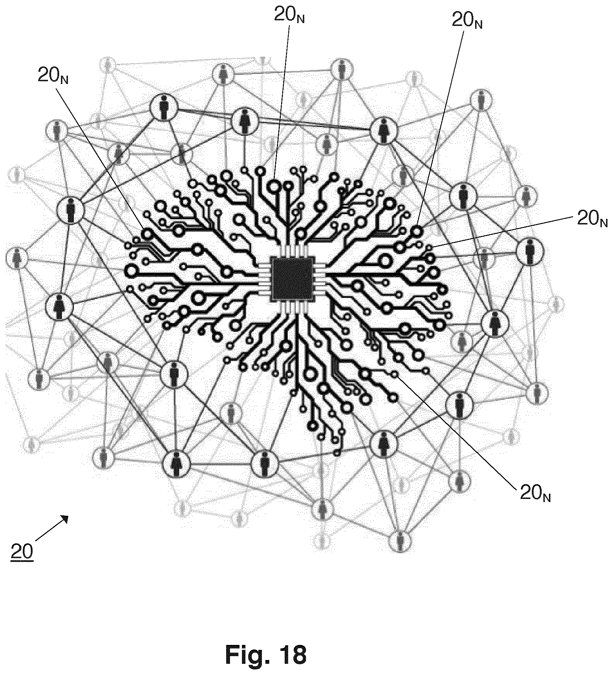

Die Einrichtung 10, in Fig. 1 prinzipiell vereinfacht dargestellt, dient bei der Untersuchung eines Patienten 11 (vgl. Fig. 2) zur Früherkennung des Vorliegens einer Koronaren Herzkrankheit (KHK) oder einer Herzrhythmusstörung (HRS), und ist nicht Teil der Erfindung. Hierzu umfasst die Einrichtung 10 vier Sensoren bzw. Elektroden S1, S2, S3, S4, einen datentechnischen Filter 16, eine Auswerteeinrichtung 18 und ein System 20 basierend auf Künstlicher Intelligenz (KI). Die Auswerteeinrichtung 18 ist mit einem (nicht gezeigten) Speicherelement ausgestattet, so dass darin Messsignale bzw. -werte zumindest kurzzeitig gespeichert werden können. Das KI-System 20 kann zumindest ein neuronales Netz $20_N$ (vgl. auch Fig. 18) oder eine Mehrzahl solcher neuronalen Netze aufweisen bzw. aus solchen neuronalen Netzen gebildet sein.

Die Sensoren S1-S4 sind signaltechnisch (z.B. über eine Kabelverbindung, oder über eine drahtlose Funkstrecke) derart an die Einrichtung 10 angeschlossen, dass deren Messwerte zunächst den Filter 16 durchlaufen und anschließend in die Auswerteeinrichtung 18 gelangen. Die Auswerteeinrichtung 18 ist datentechnisch mit dem KI-System 20 verbunden, derart, dass die Messgrößen, die mittels der Auswerteeinrichtung 18 geeignet aufbereitet bzw. in orthogonalisierte Messgrößen auf Grundlage der Vektorkardiographie überführt werden, wie nachstehend noch gesondert ist, in das KI-System 20 eingegeben werden können. Dies erfolgt zum Zwecke einer Diagnose für den Patienten 11, der unter Verwendung der Einrichtung 10 untersucht wird.

Fig. 2 zeigt - zum verbesserten Verständnis der Erfindung - einen Patienten 11, nämlich in einer Ansicht von vorne (Fig. 2a) und in einer Ansicht von hinten (Fig. 2b). Am Körper 14 des Patienten 11 sind insgesamt vier Ableitungspunkte vorgesehen, nämlich ein erster Ableitungspunkt E1, ein zweiter Ableitungspunkt E2, ein dritter Ableitungspunkt E3 und ein vierter Ableitungspunkt E4. Die Ableitungspunkte E1, E3 und E4 befinden sich jeweils im Brustbereich des Patienten 11, wobei der Ableitungspunkt E2 sich im Rückenbereich des Patienten 11 befindet. In Bezug auf diese vier Ableitungspunkte E1 bis E4 ist zu beachten, dass der erste Sensor S1 an dem ersten Ableitungspunkt E1, der zweite Sensor S2 an dem zweiten Ableitungspunkt E2, der dritte Sensor S3 an dem dritten Ableitungspunkt E3 und der vierte Sensor S4 an dem vierten Ableitungspunkt E4 am Körper 14 des Patienten 11 angebracht werden. Zwischen diesen Ableitungspunkten werden jeweils Potentialdifferenzen gemessen, wie nachstehend noch gesondert erläutert. Bezüglich weiterer Details zu den Positionen dieser einzelnen Ableitungspunkte E1-E4 am Körper 14 des Patienten wird auf die Offenbarung gemäß EP 86 429 B1, auf deren Inhalt hiermit in vollem Umfang Bezug genommen wird.

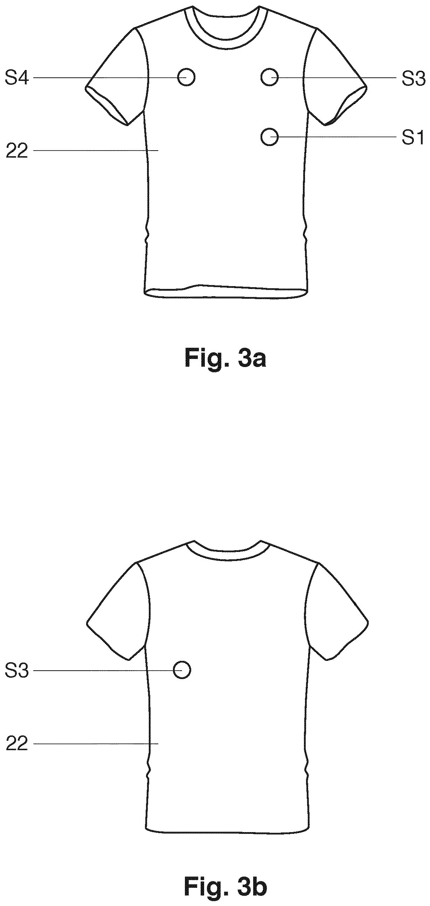

In der Fig. 3 ist vereinfacht ein T-Shirt 22 gezeigt. Die vorstehend genannten Sensoren S1-S4 der Einrichtung 10 können in das T-Shirt 22 integriert sein, zum Beispiel durch ein Verweben in dessen textiler Struktur. Ein solches T-Shirt 22 führt zu dem Vorteil, dass ein Patient 11 zur Vorbereitung einer Untersuchung lediglich dieses T-Shirt 22 anzieht bzw. überstreift, wobei dann die in das T-Shirt 22 integrierten Sensoren S1-S4 automatisch in ihre bestimmungsgemäße Position angrenzend zu den vier Ableitungspunkten E1-E4 gelangen. Durch den Einsatz eines solchen T-Shirts 22 erübrigt sich ein zeitaufwendiges und ggf. fehlerträchtiges manuelles Anlegen der einzelnen Sensoren S1-S4 am Körper 14 des Patienten 11. Alternativ zu dem T-Shirt 22 kann auch ein (nicht gezeigter) Brustgurt verwendet werden, an dem die Sensoren bzw. Elektroden S1-S4 angebracht sind.

Zur Untersuchung eines Patienten 11 bzw. zur Gewinnung eines Probedatensatzes zum Training des KI-Systems 20 werden die vier Sensoren S1-S4 an den zugeordneten vier Ableitungspunkten E1-E4 am menschlichen Körper 14 positioniert. Im Anschluss daran werden im Ruhezustand des Patienten 11 mit Hilfe der in Position gebrachten Sensoren S1-S4 EKG-Signale am Herzen 12 des Patienten 11 aufgenommen. Die EKG-Signale werden sodann durch den Filter 16 geeignet gefiltert, und anschließend in der Auswerteeinrichtung 18 in orthogonalisierte Messgrößen (in die Achsen x, y, z) nach einem orthogonalen System transformiert.

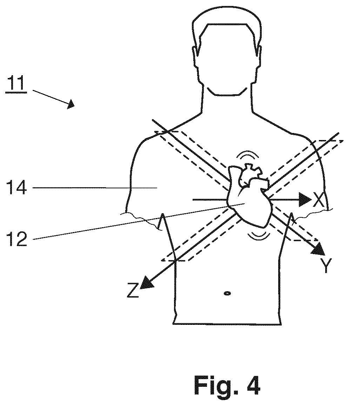

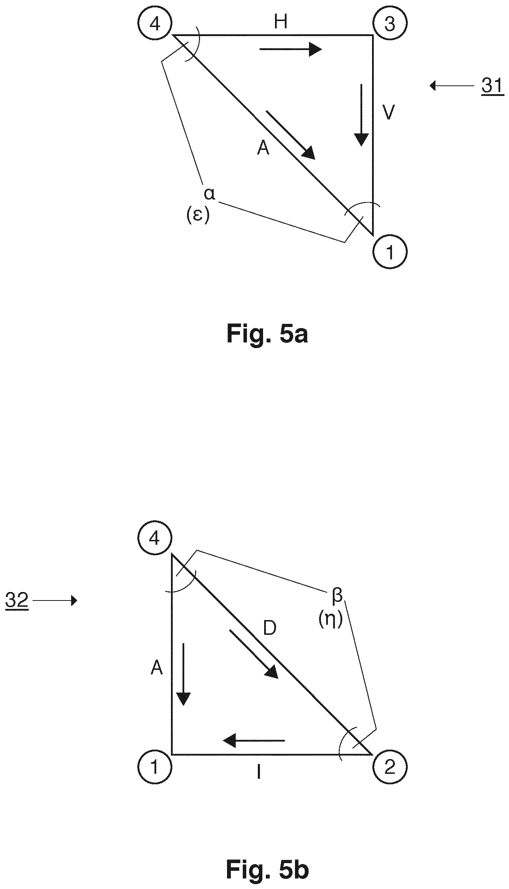

In der Fig. 4 sind die Achsen x, y, z nach einem orthogonalen System veranschaulicht, in Bezug auf den Körper 14 eines Patienten 11 und dessen Herz 12. Diesbezüglich wird auch auf die Fig. 5 verwiesen, wobei in Fig. 5a schematisch ein erstes Dreieck 31 veranschaulicht ist, welches zwischen den Ableitungspunkten E1, E3 und E4 gebildet wird, und in Fig. 5b ein zweites Dreieck 32 veranschaulicht ist, welches zwischen den Ableitungspunkten E1, E2 und E4 gewählt ist. Die Bedeutung dieser beiden Dreiecke 31, 32 wird nachfolgend noch gesondert erläutert.

Wie bereits erläutert, werden zwischen den einzelnen Ableitungspunkten E1-E4 jeweils Potentialdifferenzen erfasst. Im Einzelnen sind dies eine anteriore Ableitung A zwischen dem ersten Ableitungspunkt E1 und dem vierten Ableitungspunkt E4, eine dorsale Ableitung D zwischen dem zweiten Ableitungspunkt E2 und dem vierten Ableitungspunkt E4, eine horizontale Ableitung H zwischen dem dritten Ableitungspunkt E3 und dem vierten Ableitungspunkt E4, eine vertikale Ableitung V zwischen dem ersten Ableitungspunkt E1 und dem dritten Ableitungspunkt E3, und schließlich eine inferiore Ableitung I zwischen dem ersten Ableitungspunkt E1 und dem zweiten Ableitungspunkt E2. Aus dem ersten Ableitungspunkt E1, dem dritten Ableitungspunkt E3 und dem vierten Ableitungspunkt E4 wird - ausweislich der Darstellung in Fig. 5a - ein erstes Dreieck 31 gebildet, wobei aus dem ersten Ableitungspunkt E1, dem zweiten Ableitungspunkt E2 und dem vierten Ableitungspunkt E4 - ausweislich der Darstellung in Fig. 5b - ein zweites Dreieck 32 gebildet wird. Die genannten Ableitungen sind mit ihren Bezeichnungen A, D, H, V und I ebenfalls in den Darstellungen von Fig. 5a und Fig. 5b gezeigt. Für weitere Zusammenhänge in Bezug auf diese Ableitungen darf an dieser Stelle auf den Inhalt der EP 86 429 B1 verwiesen werden.

Die Bedeutung der ersten und zweiten Dreiecke 31, 32 ist nachfolgend noch an anderer Stelle im Zusammenhang mit einem sog. "Korrektur-Verfahren" gemäß der vorliegenden Erfindung gesondert erläutert.

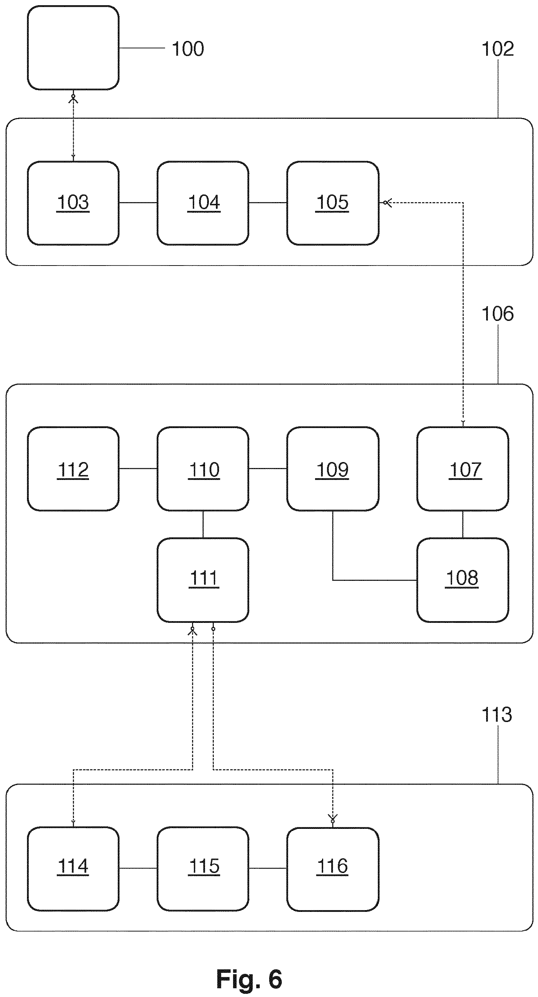

Bei Durchführung einer EKG-Messung gelangen die elektrischen Messwerte der vorstehend genannten Ableitungen A, D, H, V und I in die Einrichtung 10 und werden darin, wie oben bereits erläutert, entsprechend weiter verarbeitet. Unter Bezugnahme auf die Fig. 6 werden nachfolgend weitere Einzelheiten der Einrichtung 10 und deren Einbindung in eine Gesamtarchitektur zur Durchführung der vorliegenden Erfindung erläutert. Hierzu im Einzelnen: Die Architektur gemäß Fig. 6 sieht folgende Komponenten vor: Sensoreinrichtung 100, Datenaufzeichner 102, Cardisio®-Vorrichtung 106 und Server 113. Der Datenaufzeichner 102 umfasst einen Signalempfänger 103, einen Signalkonverter 104 und einen Signalspeicher 105. Die Cardisio-Vorrichtung 106 umfasst einen Signalleser 107, einen Vektordaten-Generator 108, einen Vektordaten-Speicher 109, einen Vektordaten-Auswerter 110, einen Vektordaten-Synchronisierer 111 und eine Vektordaten-Anzeige 112. Der Server umfasst 113 einen Vektordaten-Speicher 114, einen Vektordaten-Analysator 115 und einen Vektordaten-Auswerte-Speicher 116.

Die Sensoreinrichtung 100 wird durch die vorstehend genannten vier Sensoren bzw. Elektroden S1-S4 der Einrichtung 10 ausgebildet.

Die Sensoreinrichtung 100 und der Datenaufzeichner 102 bilden die Komponenten bzw. Bauteile der Einrichtung 10, die zur Messung bzw. Aufnahme der EKG-Signale dienen. Hiermit werden die analogen EKG-Signale empfangen, verarbeitet und geeignet in digitale Signale transformiert. Wie bereits erläutert, können die digitalen Signale zumindest kurzzeitig in dem Speicherelement der Auswerteeinrichtung 18 - vorliegend in Form des Signalspeichers 105 - gespeichert werden.

Die Cardisio-Vorrichtung 106 liest die digitalen Signale von dem Datenaufzeichner 102, zwecks einer Bestimmung von Vektordaten auf Grundlage der digitalen Signale mittels des Vektordaten-Generators 108. Die hierbei erzeugten Vektordaten werden anschließend in dem Vektordaten-Speicher 109 gespeichert. Ausgehend hiervon erzeugt der Vektordaten-Auswerter 110 eine Darstellung dieser Vektordaten z.B. in Form einer dreidimensionalen Kurve, wobei diese Darstellung dann mittels der Vektordaten-Anzeige 112 gezeigt bzw. anschaulich gemacht wird.

Die Architektur gemäß Fig. 6 veranschaulicht des Weiteren, dass innerhalb der Cardisio-Vorrichtung 106 der Vektordaten-Auswerter 110 signaltechnisch mit dem Vektordaten-Synchronisierer 111 verbunden ist, und Letzterer über eine Signalstrecke bzw. -verbindung an den Server 113 angeschlossen ist. Hierdurch können die erzeugten Vektordaten über den Vektordaten-Synchronisierer 111 zunächst in den Vektordaten-Speicher 114 auf dem Server 113 eingelesen werden. Im Anschluss daran ist es mittels des Vektordaten-Analysators 115 möglich, eine gezielte Auswertung von größeren Datenmengen bzw. der erzeugten Vektordaten vorzunehmen und diesbezüglich statistische Analysen durchzuführen. Schlussendlich werden die manuellen und/oder automatischen Auswertungen der Datensätze in dem Vektordaten-Auswerte-Speicher 116 gespeichert, wobei ausgehend hiervon diese Auswertungen wieder auf die Vorrichtung 106 hochgeladen werden können, z.B. zwecks einer Darstellung auf bzw. mit der Vektordaten-Anzeige 112.

Für die anhand der Fig. 6 erläuterten Komponenten und Bauteile der Sensoreinrichtung 100, des Datenaufzeichners 102, der Cardisio-Vorrichtung 106 und des Servers 113 versteht sich, dass diese jeweils Teile der Einrichtung 10 (vgl. Fig. 1) bilden. Insbesondere der Vektordaten-Auswerte-Speicher 116 ist Teil des KI-Systems 20 bzw. eines neuronalen Netzes $20_N$, wobei hierin bereits bekannte Befunddaten von Vergleichspatienten gespeichert sind, bezüglich derer eine eindeutige Diagnose ("gesund" oder "krank") bekannt ist.

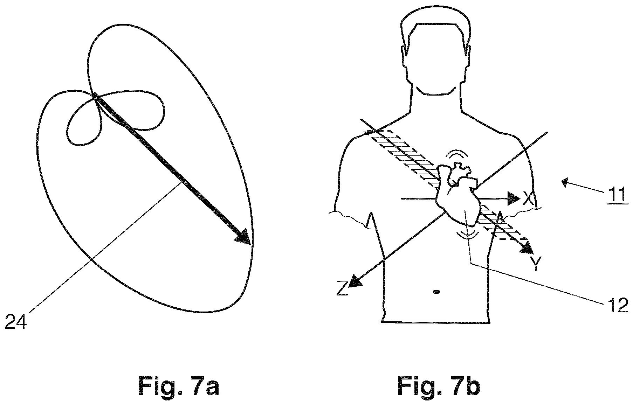

Auf Grundlage der EKG-Messdaten, die mithilfe der vier Sensoren S1-S4 am Herzen 12 eines Patienten 11 aufgenommen werden, kann durch die Auswerteeinrichtung 18 ein Raumvektor 24 erzeugt werden, der die elektrische Aktivität des Herzens 12 abbildet. Konkret bildet dieser Raumvektor 24 den zeitlichen Verlauf des Summensektors des elektrischen Feldes des Herzens 12 und weist eine der Feldrichtung entsprechende Richtung und eine dem Potenzial entsprechende Länge auf. Ein solcher Raumvektor 24 ist beispielhaft in der Fig. 7a gezeigt, der vorzugsweise in einem orthogonalen System mit den Achsen x, y, z abgebildet wirdgebildet wird. Fig. 7b zeigt nochmals - in gleicher Weise wie Fig. 4 - in vereinfachter Weise das Herz 12 eines Patienten 11, in Verbindung mit den Achsen x, y, z.

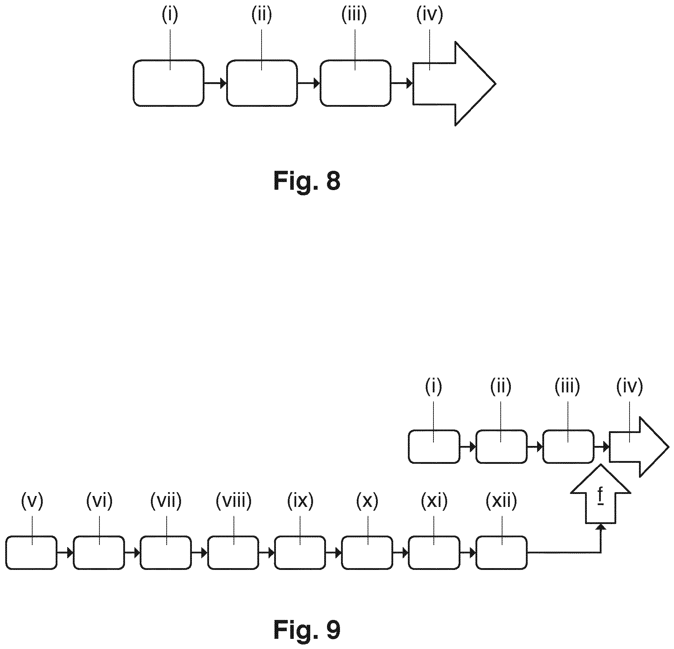

Nachstehend ist ein Verfahren, welches nicht Teil der Erfindung ist, unter Bezugnahme auf die Fig. 8 erläutert, die ein Ablaufdiagramm von Schritten eines solchen Verfahrens zeigt. Hierzu im Einzelnen:
Zu Beginn des Verfahrens werden die Sensoren S1-S4 der vorstehend erläuterten Einrichtung 10 (vgl. Fig. 1) am Oberkörper eines Patienten 11 angebracht, nämlich in Entsprechung der vier Ableitungspunkte E1, E2, E3 und E4 (vgl. Fig. 2a, Fig. 2b). Zu diesem Zweck kann das T-Shirt 22 von Fig. 3 eingesetzt werden. In dieser Weise werden dann am Herzen 12 des Patienten 11 in nicht-invasiver Weise EKG-Signale aufgenommen, nämlich im Ruhezustand des Patienten 11. Dies entspricht einem Schritt (i) des Verfahrens gemäß Fig. 8.

Im Anschluss daran werden in einem Schritt (ii) des Verfahrens von Fig. 8 die aufgenommenen EKG-Signale filtertechnisch aufbereitet, nämlich, wie unter Bezugnahme auf Fig. 1 erläutert, durch den Filter 16. Ein solches Filtern dient dazu, hochfrequentes Rauschen und niederfrequente Störungen (z.B. verursacht durch die Atmung des Patienten) zu eliminieren. Beispielhafte Filtertypen sind Kerbfilter, Hochpassfilter, Sarvitzky-Golay-Tiefpassfilter.

In dem hiernach folgenden Schritt (iii) des Verfahrens von Fig. 8 werden dann die gefilterten EKG-Signale mittels der Auswerteeinrichtung 18, die signaltechnisch an den Filter 16 angeschlossen ist, auf Grundlage der Vektorkardiographie in orthogonalisierte Messgrößen überführt. Hierbei werden signifikante Bereiche in dem Signal eines Herzschlags lokalisiert, z.B. Beginn und Ende des QRS-Komplexes, Beginn, Maximum und Ende der T-Welle. Zur Bestimmung der tatsächlichen Zeitpunkte der physiologischen Extrempunkte ist es vorteilhaft, wenn die genannten Ableitungen A, D, H, I und V, die zwischen den Ableitungspunkten E1-E4 (vgl. Fig. 2a, Fig. 2b) gewonnen werden, jeweils in Kugelkoordinaten transformiert werden.

Schließlich werden in einem weiteren Schritt (iv) des Verfahrens nach Fig. 8 die orthogonalisierten Messgrößen in das KI-System 20 bzw. ein neuronales Netz $20_N$ eingegeben. Hierbei ist zu beachten, dass das KI-System 20 auf bereits bekannten Befunddaten von Vergleichspatienten basiert, für die - wie eingangs bereits erläutert - eine eindeutige Kenntnis in Bezug auf deren Gesundheitszustand ("gesund" oder "krank") vorliegt. Auf Grundlage dessen kann dann mittels des Vektordaten-Analysators 115 und des Vektordaten-Auswerte-Speichers 116 durch einen Vergleich der eingegebenen orthogonalisierten Messgrößen mit den Befunddaten der Vergleichspatienten innerhalb des KI-Systems 20 eine Diagnose für den untersuchten Patienten 11 erstellt werden.

Für den Schritt (i) des Verfahrens von Fig. 8 empfiehlt sich, dass eine nicht-invasive Aufnahme von EKG-Signalen am Herzen 12 des Patienten 11 an genau den vier Ableitungspunkten E1-E4 erfolgt, die vorstehend unter Bezugnahme auf die Fig. 2a und Fig. 2b erläutert worden sind.

Mittels einer vorteilhaften Weiterbildung bzw. Ergänzung des Verfahrens von Fig. 8 ist es möglich, dass das KI-System 20 bzw. das neuronale Netz $20_N$ vor dem Schritt (iv) trainiert wird, nämlich durch eine Eingabe von spezifischen Lernwerten f. Dies ist nachfolgend anhand der Fig. 9 gezeigt und im Einzelnen erläutert:
Die Anzahl der spezifischen Lernwerte f, mit denen das KI-System 20 bzw. das neuronale Netz $20_N$ vor dem Schritt (iv) trainiert wird, kann zwischen 10 und 30 liegen, und z.B. den Wert von 20 annehmen. Diese spezifischen Lernwerte f werden durch folgende Schrittabfolge bestimmt:

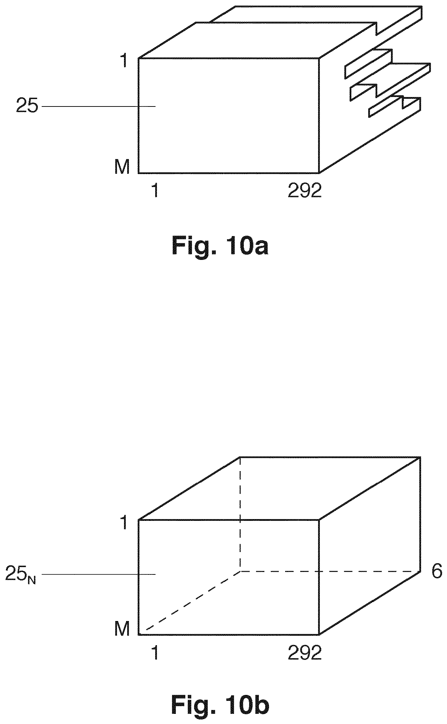

(v) Bereitstellen von Messgrößen von einer Menge M (vgl. Fig. 10) von Patienten 11 mit einem bekannten Befund, wobei diese Messgrößen auf Grundlage der Vektorkardiographie orthogonalisiert sind,

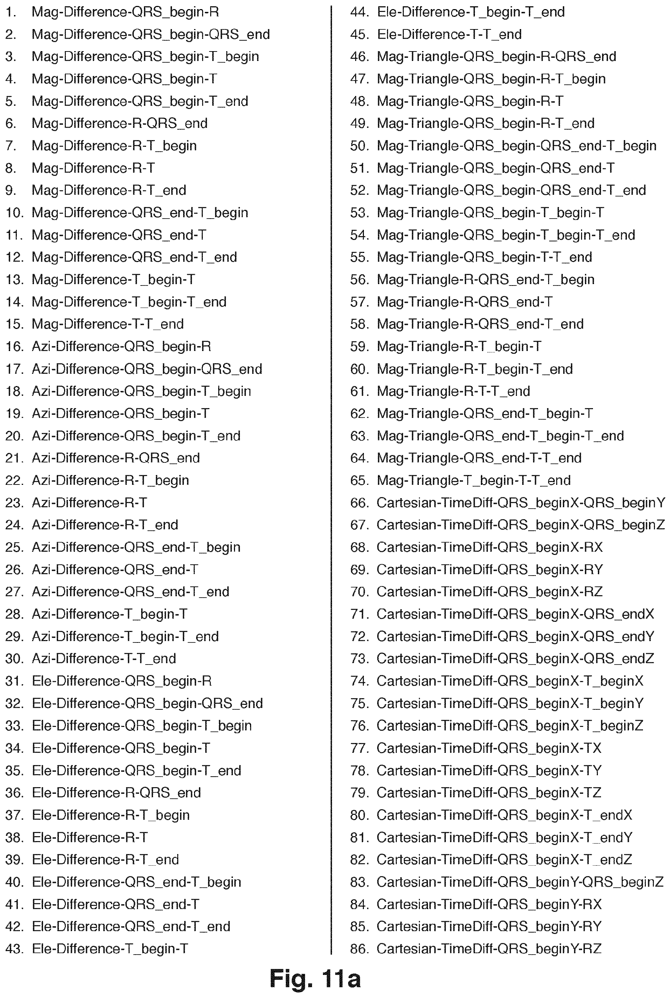

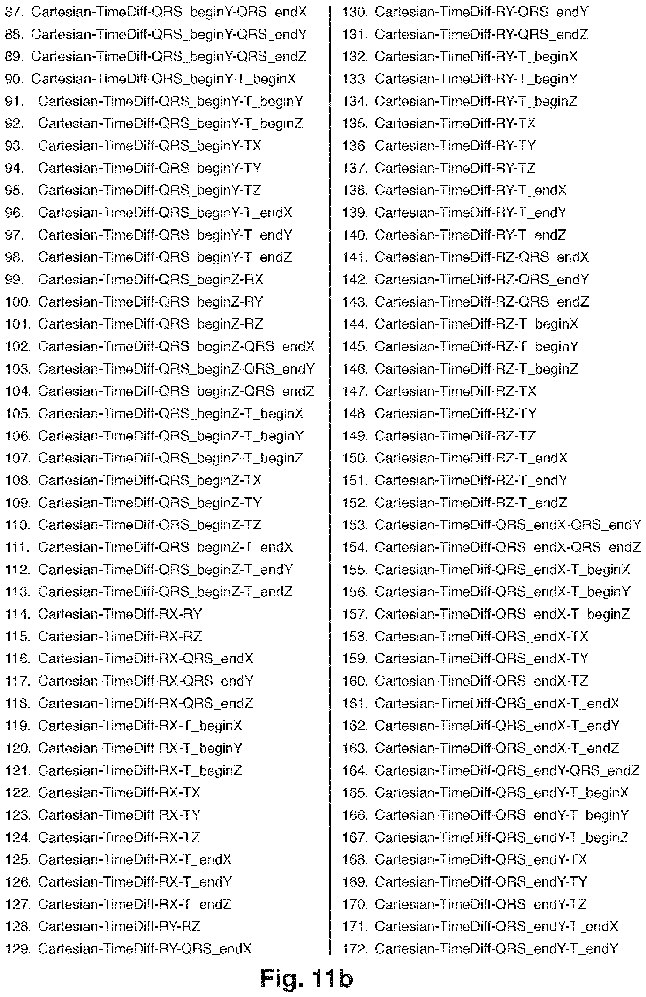

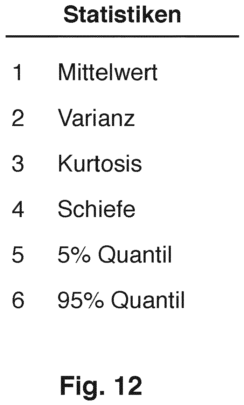

(vi) Bereitstellen einer Mehrzahl von Zeitreihen-Parametern (vgl. Fig. 11a und Fig. 11b) und zumindest einem statistischen Verfahren (vgl. Fig. 12),

(vii) Bilden einer 3D-Matrix 25 (vgl. Fig. 10a), wobei die orthogonalisierten Messgrößen der Menge (M) von Patienten die Zeilen, die Zeitreihen-Parameter die Spalten und die Zeitreihenlänge die Tiefe dieser Matrix (25) definieren, wobei im Falle skalarer Parameter die Tiefe gleich eins ist,

(viii) Klassifizieren aller Wertepaare der 3D-Matrix (25) nach dem Prinzip der "Area-under-Curve" (AUC)-Berechnung,

(ix) Auswählen eines Wertepaars aus der Menge gemäß Schritt (viii) mit dem höchsten AUC-Wert,

(x) Überprüfen eines weiteren Wertepaars aus der Menge gemäß Schritt (viii), und Auswählen dieses Wertepaars, falls hierfür ein Schwellenwert für eine Korrelation mit dem Wertepaar von Schritt (ix) betragsmäßig kleiner als $1{,}65/\sqrt{N}$ ist, mit N= Anzahl der Datenpunkte bzw. Parameter-Statistiken (Patienten) gemäß Schritt (vi)

(xi) Wiederholen des Schritts (x) für ein weiteres Wertepaar aus der Menge gemäß Schritt (viii), und Auswählen dieses Wertepaars, falls ein Schwellenwert für eine Korrelation mit den zuvor ausgewählten Wertepaaren jeweils betragsmäßig kleiner als $1{,}65\sqrt{N}$ ist, und

(xii) Wiederholen der Schritte (ix) bis (xi) solange, bis eine vorbestimmte Anzahl von z.B. 20 Wertepaaren erreicht ist, die dann als spezifischen Lernwerte f definiert und zum Trainieren des KI-Systems (20) darin eingegeben werden.

Die vorstehend genannten Schritte (v) bis (xii) der Weiterbildung des Verfahrens gemäß Fig. 9 sind in dem zugehörigen Ablaufdiagramm jeweils vereinfacht durch Blöcke symbolisiert. Hierbei ist der Pfeil "f", der nach dem Schritt (xii) von unten in den Ablauf zwischen den Schritten (iii) und (iv) einmündet, so zu verstehen, dass die im Schritt (xii) definierten vorbestimmte Anzahl von spezifischen Lernwerten f in das KI-System 20 bzw. das neuronale Netz $20_N$ eingegeben werden.

In Bezug auf die vorteilhafte Weiterbildung des Verfahrens gemäß Fig. 9 darf erläuternd darauf hingewiesen werden, dass in Schritt (v) die Messgrößen der Menge M von Patienten 11 in Form von Zeitreihen vorzugsweise in Millisekunden oder in Form von Herzschlägen bereitgestellt werden. Die Bildung der 3D- Matrix 25 gemäß Schritt (vii) ist symbolisch in der Fig. 10a dargestellt. Die Menge M aller Patienten ist als Ordinate aufgetragen und kann, z.B. von oben nach unten gesehen, zunächst in eine Gruppe von gesunden Patienten (z.B. ohne KHK-Befund) und dann in eine Gruppe von kranken Patienten (z.B. mit KHK-Befund) unterteilt sein. Die 3D-Matrix umfasst die Menge M der insgesamt 284 Zeitreihen, sowie der skalaren persönlichen Parameter, welche in den Fig. 11a - 11d gezeigt sind. In das Training des neuronalen Netzes gehen jedoch nur die 282 Zeitreihen Parameter ein.

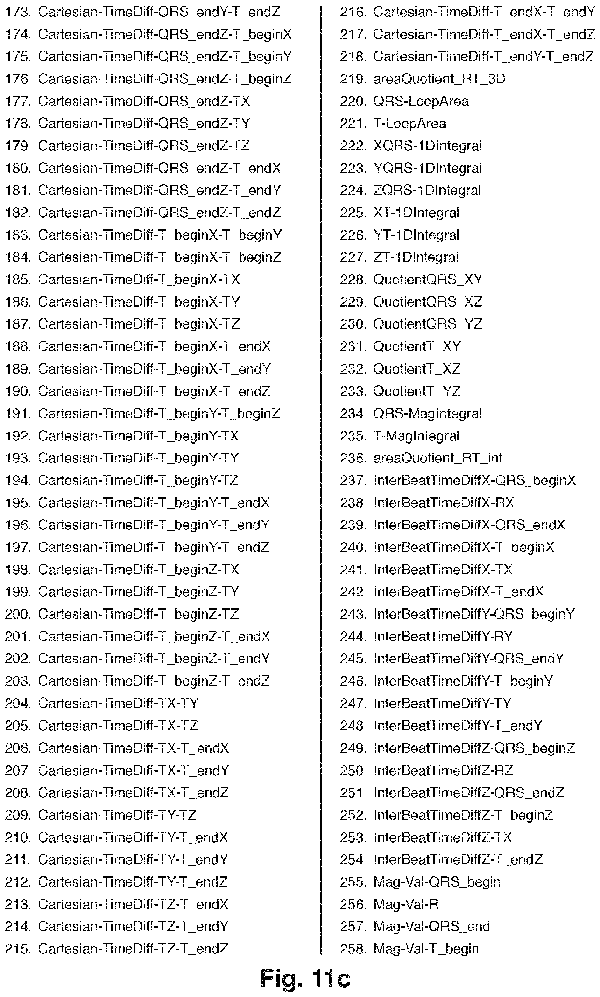

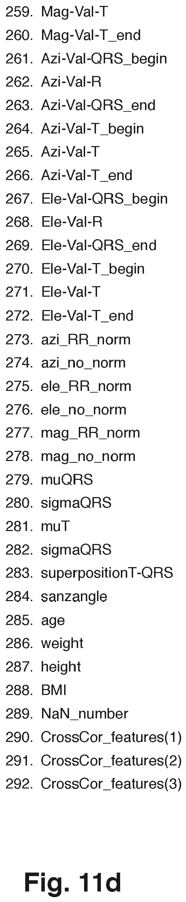

In Bezug auf die 292 Parameter, die in den Fig. 11a, 11b, 11c und 11d dargestellt sind, wird gesondert darauf hingewiesen, dass es sich hierbei um 284 Zeitreihen-Parameter handelt. 8 persönliche skalare Parameter, die sich auf einen jeweils untersuchten Patienten beziehen werden über den Satz von Bayes für die Wahrscheinlichkeitsberechnungen berücksichtigt.

An dieser Stelle wird gesondert darauf hingewiesen, dass bei dem vorstehend genannten Verfahren, in dessen Schritten (x) und (xi), die einzelnen Schwellenwerte für die jeweiligen Korrelationen mit dem Wertepaar von Schritt (ix) keinen konstanten festen Wert annehmen, sondern jeweils abhängig sind von der Anzahl von Herzschlägen bzw. von der Mehrzahl von Zeitreihen-Parametern gemäß Schritt (vi). Somit werden für kurze Zeitreihen (und damit kleinere Werte von N) höhere Korrelationen zugelassen, und umgekehrt.

Fig. 10b zeigt die 3D-Matrix in einer normierten Version mit einheitlicher Tiefe, die dadurch erreicht wird, dass deren Spalten (= Patienten-Daten) und Zeilen (= Zeitreihen-Parameter) mit einer Mehrzahl von statistischen Verfahren verknüpft bzw. verrechnet werden, von denen beispielhaft sechs Verfahren in Fig. 12 gezeigt sind, nämlich:

- Mittelwert
- Varianz
- Kurtosis,
- Schiefe,
- 5%-Quantil,
- 95%-Quantil.

Die mögliche Anwendung dieser sechs Verfahren ist in der Darstellung von Fig. 10b durch den Eintrag der "6" (im Bildbereich rechts) angedeutet. Hierzu wird darauf hingewiesen, dass im Schritt (vii) zumindest eines dieser statistischen Verfahren, oder auch mehrere solcher Verfahren, angewendet werden können, um damit sowohl die Tiefe der 3D- Matrix 25 zu definieren und ggf. eine einheitliche Tiefe zur Realisierung einer normierten Matrix $25_N$ erreicht wird.

Des Weiteren darf für das Verfahren gemäß Fig. 9 erläuternd darauf hingewiesen werden, dass in Schritt (viii) die AUC-Berechnung empirisch oder nach dem Prinzip der Johnson-Verteilung erfolgen kann. Das Prinzip der Johnson-Verteilung ist per se Stand der Technik bekannt, könnte jedoch mit der vorliegenden Erfindung erstmals im Zusammenhang mit der Auswertung von Patientendaten bzw. EKG-Signalen zwecks einer Früherkennung des Vorliegens einer KHK und/oder einer HRS eingesetzt werden.

Wie bereits erläutert, ist es zur Auswertung der Daten zwecks Erstellung einer Diagnose von Vorteil, wenn das KI-System 20, in das die spezifischen Lernwerte f eingegeben werden, zumindest ein neuronales Netz $20_N$, oder eine Mehrzahl von solchen Netzen $20_N$, aufweist.

Mittels der vorliegenden Erfindung ist es möglich, wie erläutert einerseits ein Verfahren zur Früherkennung des Vorliegens einer KHK und/oder einer HRS eines zu untersuchende Patienten 11 durchzuführen, wie es anhand des Ablaufdiagramms von Fig. 8 gezeigt und erläutert worden ist. Andererseits ist es möglich, zur Verbesserung der Diagnose des Patienten 11 das KI-System 20 (bzw. ein neuronales Netz $20_N$) vor der eigentlichen Messung einem Training zu unterziehen, wie es anhand des Ablaufdiagramms von Fig. 9 gezeigt und erläutert worden ist. Ein solches Training wird auf Grundlage der Daten von solchen Patienten durchgeführt, von denen eine eindeutige Kenntnis in Bezug auf deren Gesundheitszustand ("gesund" oder "krank") vorliegt. Insoweit ist ein solches Training im Sinne der vorigen Erfindung auch als "überwachtes Training" (*supervised learning*) zu verstehen.

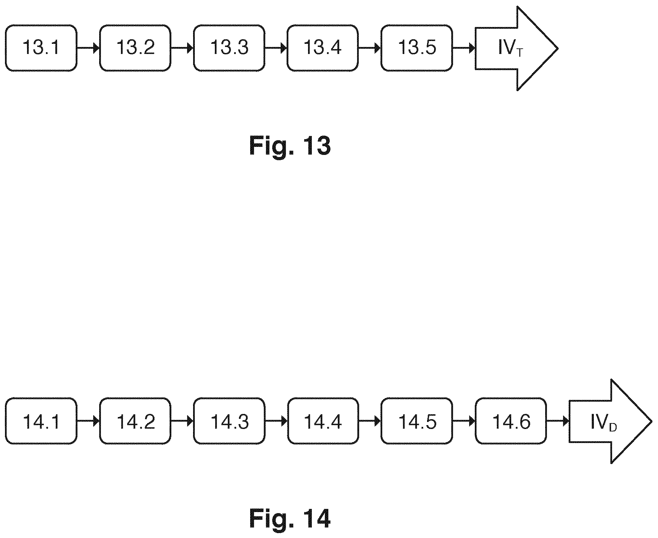

Diese beiden Möglichkeiten, nämlich sowohl ein vorgeschaltetes Training für das KI-System 20 (bzw. ein neuronales Netz $20_N$) als auch die eigentliche Durchführung der Vermessung eines Patienten 11 zwecks Erstellung einer gewünschten Diagnose, sind nachfolgend nochmals in den Ablaufdiagrammen der Fig. 13 und Fig. 14 dargestellt. Das Ablaufdiagramm von Fig. 13 zeigt mit seinen Blöcken 13.1 - 13.5 eine Schrittabfolge zwecks eines Trainings des KI-Systems 20. Ein solches Training dient zur Optimierung der eigentlichen Untersuchung bzw. Diagnose eines Patienten 11, die durch die Schrittabfolge des Ablaufdiagramms von Fig. 14 veranschaulicht ist.

Im Ablaufdiagramm von Fig. 13 entspricht der Schritt 13.1 im Wesentlichen dem Schritt (i) von Fig. 8, wobei der Schritt 13.2 im Wesentlichen dem Schritt (ii) von Fig. 8 entspricht. Gleiches gilt auch für den Schritt 13.3, der im Wesentlichen dem Schritt (iii) von Fig. 8 entspricht. Zur Vermeidung von Wiederholungen darf deshalb für diese Schritte 13.1, 13.2 und 13.3 auf die Erläuterungen zu Fig. 8 verwiesen werden.

Der nun folgende Schritt 13.4 im Ablaufdiagramm von Fig. 13 entspricht einer Parameter-Gewinnung (*"parameter extraction")*, bei der - in Entsprechung der Schritte (v) bis (vii) von Fig. 9 - Zeitreihen-Parameter mit den Messgrößen einer Menge M von Patienten 11 verknüpft bzw. verrechnet werden. Auch hierbei erweist es sich als Vorteil, wenn dies auf Grundlage von Kugelkoordinaten erfolgt, in welche die Ableitungen A, D, H, I und V geeignet transformiert worden sind.

Der anschließende Schritt 13.5 bezweckt eine Merkmals-Bestimmung (*"feature evaluation")* und entspricht im Wesentlichen eine Abfolge der Schritte (viii) bis (xii) von Fig. 9, die vorstehend bereits genannt und erläutert worden sind. Dies bedeutet, dass mit dieser Merkmals-Bestimmung gemäß Schritt 13.5 die spezifischen Lernwerte f bestimmt bzw. ermittelt werden.

Im Anschluss daran werden dann bei dem Ablaufdiagramm von Fig. 13, nämlich im Schritt $IV_T$, die spezifischen Lernwerte f in das KI-System 20 (bzw. in ein neuronales Netz $20_N$) eingegeben, in Entsprechung des Schritts (iv) von Fig. 8. Im Ergebnis wird damit durch die Eingabe der spezifischen Lernwerte f das KI-System 20 geeignet trainiert. An dieser Stelle darf nochmals darauf hingewiesen werden, dass es im Rahmen dieses Trainings von großem Vorteil ist, dass die Anzahl der spezifischen Lernwerte f relativ gering ist, und z.B. den Wert 20 annehmen kann. Alternativ hierzu kann die Anzahl der spezifischen Lernwerte f auch kleiner oder größer 20 sein, und z.B. 15 oder 25 betragen. Jedenfalls ist in Bezug auf das hier diskutierte Training des KI-Systems 20 mit Hilfe dieser Lernwerte f zu verstehen, dass diese stets anhand der Daten eines Patienten 11 gewonnen werden, für den eine eindeutige Kenntnis in Bezug auf deren Gesundheitszustand ("gesund" oder "krank") vorliegt.

Das Ablaufdiagramm von Fig. 14 veranschaulicht mit seiner Schrittabfolge dann die tatsächlich durchgeführte Diagnose für einen Patienten 11. Hierbei entsprechen die Schritte 14.1, 14.2 und 14.3 im Wesentlichen den Schritten 13.1-13.3, so dass zur Vermeidung von Wiederholungen auf die Erläuterung zu den Schritten 13.1-13.3 verwiesen werden darf.

Der Schritt 14.5 beim Ablaufdiagramm von Fig. 14 sieht eine Merkmals-Auswahl (*"feature selection")* vor - hierbei werden nur die Daten eines untersuchten Patienten 11 auf Grundlage der aufgenommenen EKG-Signale verwendet, die den zuvor gemäß Schritt 13.5 bestimmten spezifischen Lernwerten f entsprechen. Ein Vergleich dieser ausgewählten Merkmale bzw. Werte mit den vorbestimmten spezifischen Lernwerten f erfolgt dann in Schritt 14.6 ("trained network"). Im Anschluss hieran wird dann im Schritt $IV_D$ mit Hilfe des trainierten KI-Systems 20 für den Patienten 11 die eigentliche Diagnose $IV_D$ gestellt, in Entsprechung des Schritts (iv) von Fig. 8.

Die vorstehend erläuterte Diagnose eines Patienten 11, die mit einer Einrichtung 10 von Fig. 1 und durch ein Verfahren nach dem Ablaufdiagramm von Fig. 8 bzw. Fig. 14 durchgeführt wird, als auch das Training eines KI-Systems 20 (bzw. eines neuronalen Netzes 20N), das nach dem Ablaufdiagramm von Fig. 9 bzw. Fig. 13 durchgeführt wird, beruhen stets darauf, dass die Sensoren S1-S4 an dem Körper des Patienten 11 angebracht werden, z.B. unter Verwendung des T-Shirts von Fig. 3. Hierbei ist es nun nach einem weiteren Verfahren, das nicht Teil der Erfindung ist, einen ggf. nicht korrekten Sitz dieser Sensoren S1-S4 am Körper des Patienten 11 zu kompensieren, so dass damit eine weiterhin realistische Diagnose für den Patienten 11 gewährleistet ist. Ein solches Verfahren, nachstehend kurz als "Korrektur-Verfahren" bezeichnet, sieht zunächst vor, dass ein auf das Herz 12 des Patienten 11 bezogener Raumvektor 24 (vgl. Fig. 7a), der den Vektor des durch die Aktivität des Herzens 12 gebildeten elektrischen Feldes darstellt, ermittelt und geeignet dargestellt wird. Hierbei werden Messwerte des Herzens 12 am Körper 14 des Patienten an den insgesamt vier Ableitungspunkten E1-E4 erfasst, wie vorstehend bereits im Zusammenhang mit der Fig. 2 erläutert worden ist. Bei dem soeben genannten Korrektur-Verfahren wird auf Grundlage der Ableitungen A, D, H, I und V (vgl. Fig. 5a, Fig. 5b) ein orthogonales System mit den Beziehungen

$$x = D \cos 45° - I$$

$$y = D \sin 45° + A$$

$$z = (V - H) \sin 45°$$

gebildet, wobei die Messwerte für den Patienten 11 und der daraus ermittelte Raumvektor 24 in diesem orthogonalen System x, y, z abgebildet werden.

Damit sieht dann das Korrektur-Verfahren unter Bezugnahme auf die Darstellungen in den Fig. 5a, Fig. 5b, Fig. 15 und Fig. 16 im Einzelnen folgende Schritte vor:

(a) Durchführung einer Messung an einem Patienten unter Verwendung der ersten bis vierten Ableitungspunkte E1-E4 am Körper 14 des Patienten, um damit für diesen Patienten ein Kardiogramm zu gewinnen,
(b) Extrahieren der Amplituden der R-Welle aus dem Kardiogramm von Schritt (a) für jeden Herzschlag in x- , y- und z-Richtung,
(c) Ermitteln von Mittelwerten $\mu_x$, $\mu_y$, $\mu_z$ und Standard-Abweichungen $\sigma_x$, $\sigma_y$, $\sigma_z$ der jeweils in Millivolt erfassten Amplituden aus dem Kardiogramm gemäß Schritt (b), wobei mit diesen Mittelwerten $\mu_x$, $\mu_y$, $\mu_z$ und Standard-Abweichungen $\sigma_x$, $\sigma_y$, $\sigma_z$ dann ein Berechnungsvektor 26 gebildet wird,
(d) Bilden einer Koeffizientenmatrix 28, die auf Grundlage einer Hauptachsentransformation für Messungen an Vergleichs-Patienten mit unterschiedlichen Anlageschemata gewonnen worden ist,
(e) Multiplizieren des Berechnungsvektors 26 von Schritt (c) mit der Koeffizientenmatrix 28 von Schritt (d), zur Bildung eines Ergebnisvektors 30 mit insgesamt sechs Hauptachsen $PC_1$ - $PC_6$,
(f) Extrahieren der ersten Hauptachse $PC_1$ und der zweiten Hauptachse $PC_2$ aus dem Ergebnisvektor 30 von Schritt (e), zur Bildung eines Referenzpunktes $PC_1$, $PC_2$ im Raum der ersten und zweiten Hauptachse,
(g) Ermitteln eines euklidischen Abstands des Referenzpunktes $PC_1$, $PC_2$ von einem vorbestimmten Zielpunkt $PC_1^{fit}$, $PC_2^{fit}$, der einer korrekten Position der vier Ableitungspunkte E1-E4 am menschlichen Körper 14 entspricht, und
(h) falls der Abstand des Referenzpunktes $PC_1$, $PC_2$ von dem vorbestimmten Zielpunkt $PC_1^{fit}$, $PC_2^{fit}$ größer als ein vorbestimmter Maximalwert ist: Durchführen einer Winkelkorrektur für ein aus dem ersten Ableitungspunkt E1, dem dritten Ableitungspunkt E3 und dem vierten Ableitungspunkt E4 gebildetes erstes Dreieck 31 und für ein aus dem ersten Ableitungspunkt E1, dem zweiten Ableitungspunkt E2 und dem vierten Ableitungspunkt E4 gebildetes zweites Dreieck 32, so dass damit der euklidische Abstand zwischen dem Referenzpunkt $PC_1$, $PC_2$ und dem vorbestimmten Zielpunkt $PC_1^{fit}$, $PC_2^{fit}$ durch Anpassung des orthogonalen Systems x, y, z an die geänderte Geometrie minimiert wird.

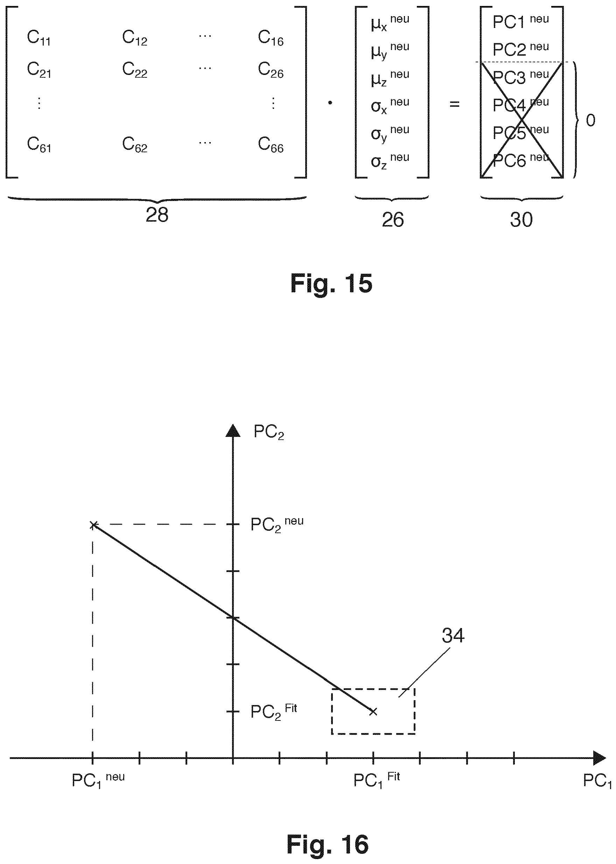

Die obigen Schritte (a) bis (h) des Korrektur-Verfahrens sind dahingehend erläutert, dass es sich bei den Amplituden, mit denen im Schritt (c) der Berechnungsvektor 26 gebildet wird, um jene Messwerte handelt, die zuvor durch die Messung gemäß Schritt (a) bzw. bei dem Verfahren von Fig. 8 in dessen Schritt (i), beim Verfahren von Fig. 13 in dessen Schritt 13.1 und/oder beim Verfahren von Fig. 14 in dessen Schritt 14.1 nicht-invasiv mit den EKG-Signalen am Herzen 12 des Patienten 11 aufgenommen werden. Der Schritt (e), wonach der Berechnungsvektor 26 mit der 6x6-Koeffizientenmatrix 28 von Schritt (d) multipliziert und daraus der Ergebnisvektor 30 resultiert, ist in der Fig. 15 veranschaulicht. Dieser Ergebnisvektor 30 liegt in Form einer 6x1-Matrix vor. Im Schritt (f) werden die dritte bis sechste Zeile des Ergebnisvektors 30, wie in Fig. 15 mit der Durchstreichung kenntlich gemacht, gestrichen bzw. für die weitere Berechnung ignoriert, womit die erste Hauptachse $PC_1$ und die zweite Hauptachse $PC_2$ aus dem Ergebnisvektor 30 extrahiert werden, zur Bildung des Referenzpunktes $PC_1$, $PC_2$. Des Weiteren ist der euklidische Abstand, der im Schritt (g) des Korrektur-Verfahrens ermittelt wird, ist in dem Diagramm von Fig. 16 veranschaulicht.

Das Diagramm von Fig. 16 zeigt auch ein beispielhaftes Zielgebiet, hier durch ein Rechteck 34 mit gestrichelten Linien symbolisiert. Diesbezüglich wird darauf hingewiesen, dass vor Durchführung des Schritts (h) der vorbestimmte

Zielpunkt PC$_1$$^{fit}$, PC$_2$$^{fit}$ aus diesem Zielgebiet ausgewählt wird, das auf Grundlage von Standard-Abweichungen einer Mehrzahl von überprüften korrekten Messungen des Zielpunktes am menschlichen Körper 14 gebildet wird.

Im Schritt (h) des Korrektur-Verfahrens können durch die hierin definierte Winkelkorrektur Anpassungswerte $\varepsilon$, $\eta$ ermittelt werden, mit denen die nicht-rechtwinkligen Winkel $\alpha$ des ersten Dreiecks 31 und die nicht-rechtwinkligen Winkel $\beta$ des zweiten Dreiecks 32 korrigiert werden können. Damit ist es möglich, eine insbesondere nicht korrekte Position des ersten Ableitungspunkts E1 am menschlichen Körper 14 zu kompensieren, um gegebenenfalls nicht-rechtwinklige Dreiecke zu berücksichtigen. In dieser Weise stellt der Anpassungswert $\varepsilon$ einen adjustierten Wert dar, der im Falle von korrekt angelegten Elektroden identisch mit dem Winkel $\alpha$ des ersten Dreiecks 31 ist. Gleiches gilt für den adjustierten Wert $\eta$ , der im Falle von korrekt angelegten Elektroden mit dem Winkel $\beta$ des zweiten Dreiecks 32 übereinstimmt. Dieser Zusammenhang ist auch grafisch in den Darstellungen von Fig. 5a und Fig. 5b gezeigt.

Die vorstehend genannten Anpassungswerte $\varepsilon$, $\eta$, die für die Winkelkorrektur von Schritt (e) verwendet werden können, lassen sich durch Minimierung des euklidischen Abstands zwischen Referenz und Zielpunkt bestimmen.

Die Koeffizientenmatrix 28, mit der in Schritt (e) der Berechnungsvektor 26 multipliziert wird, wird über eine Hauptachsentransformation einer 23x6-Matrix gebildet, die auf 23 Testmessungen und den Mittelwerten und Standard-Abweichungen der daraus ermittelten Messwerte beruht.

Das vorstehend erläuterte Korrektur-Verfahren beruht auf dem Prinzip einer Hauptachsen-Transformation, mit der im Ergebnis ein nicht korrekter Sitz von Elektroden bzw. der Sensoren S1-S4 am menschlichen Körper 14 kompensiert werden kann. Dies gilt insbesondere für die Position des Sensors S1, der dem ersten Ableitungspunkt E1 zugeordnet ist, und ist z.B. für den Fall vorteilhaft, dass die Messwerte des Herzens 12 mit dem T-Shirt 22 von Fig. 3 erfasst werden.

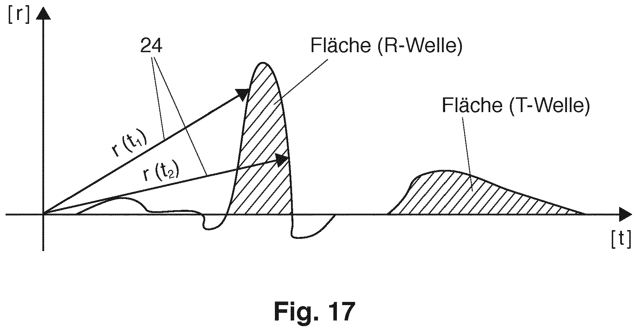

Vorstehend ist bei der Diskussion von Fig. 7a bereits darauf hingewiesen worden, dass der darin gezeigte Raumvektor 24, der anhand der Vektorkardiographie bestimmt wird, die elektrische Aktivität des Herzens 12 darstellt. Für die vorliegende Erfindung wird damit ein Herzüberwachungsverfahren definiert, bei dem ein Quotient aus den Flächen, die von einer Länge des Raumvektors 24 (= Radiusvektor) als Funktion der Zeit jeweils während der R-Welle und während der T-Welle überstrichen werden, gebildet wird, wobei anschließend dieser Quotient einer weiteren Auswertung zugeführt wird. Diese von dem Raumvektor 24 als Funktion der Zeit überstrichenen Flächen sind beispielhaft in dem Diagramm von Fig. 17 gezeigt.

Für die vorliegende Erfindung hat sich herausgestellt, dass für das untersuchte Herz 12 eines Patienten 11 eine Ischämie bzw. eine koronare Herzkrankheit (KHK) erkannt wird, falls der aus den von dem Raumvektor 24 jeweils während der R-Welle und während der T-Welle überstrichenen Flächen gebildete Quotient

$$\frac{\text{Fläche (R-Welle)}}{\text{Fläche (T-Welle)}} = a$$

außerhalb eines Intervalls zwischen den Grenzwerten $a_{0,KHK}$ und $a_{1,KHK}$ liegt.

Einen Sonderfall der vorstehend genannten Erkenntnisse bildet der Fall, dass für das untersuchte Herz 12 eine Herzrhythmusstörung (HRS) erkannt wird, falls der aus den von dem Raumvektor 24 jeweils während der R-Welle und während der T-Welle überstrichenen Flächen gebildete Quotient die Bedingung

$$\frac{\text{Fläche (R-Welle)}}{\text{Fläche (T-Welle)}} < a_{0,\ HRS}$$

oder die Bedingung

$$\frac{\text{Fläche (R-Welle)}}{\text{Fläche (T-Welle)}} > a_{1,\ HRS}$$

erfüllt.

Hinsichtlich der Grenzwerte $a_{0,KHK}$ und $a_{1,KHK}$ bzw $a_{0,HRS}$ und $a_{1,\ HRS}$, mit denen der Quotient gebildet aus der Fläche (R-Welle/Fläche (T-Welle) bei der Untersuchung des Vorliegens einer KHK und/oder HRS bei der tatsächlichen Untersuchung eines Patienten jeweils verglichen bzw. korreliert wird, darf zur Vermeidung von Wiederholungen auf die Erläuterungen im einleitenden Teil der vorliegenden Patentanmeldung verwiesen werden, wonach diese Grenzwerte auch in Abhängigkeit von einem Trainingsset bestimmt bzw. optimiert werden können.

Die verschiedenen Erkenntnisse, die für das zuletzt genannte Verfahren nach der vorliegenden Erfindung auf dem Verhältnis der Flächen, die von dem Raumvektor 24 als Funktion der Zeit jeweils während der R-Welle und während der T-Welle überstrichen werden, können selbstverständlich auch bei den zuvor genannten Verfahren angewendet bzw. berücksichtigt werden, die anhand der Ablaufdiagramme nach Fig. 8, Fig. 9, Fig. 13 bzw. Fig. 14 gezeigt und erläutert worden sind.

Bezugszeichenliste

| | |
|---|---|
| 10 | Einrichtung zur Früherkennung einer KHK oder einer HRS |
| 11 | Patient |
| 12 | Herz (des Patienten 11) |
| 14 | Körper (des Patienten 11) |
| 16 | Filter |
| 18 | Auswerteeinrichtung |
| 20 | System basierend auf Künstlicher Intelligenz (KI) |
| $20_N$ | Neuronales Netz |
| 22 | T-Shirt |
| 24 | Raumvektor |
| 25 | 3D-Matrix |
| $25_N$ | normierte 3D-Matrix |
| 26 | Berechnungsvektor |
| 28 | Koeffizientenmatrix |
| 30 | Ergebnisvektor |
| 31 | erstes Dreieck |
| 32 | zweites Dreieck |
| 34 | Zielgebiet |
| 13.1-13.5 | Schritte eines Verfahrens (vgl. Fig. 13) |
| 14.1-14.5 | Schritte eines Verfahrens (vgl. Fig. 14) |
| 100 | Sensoreinrichtung |
| 102 | Datenaufzeichner |
| 103 | Signalempfänger |
| 104 | Signalkonverter |
| 105 | Signalspeicher |
| 106 | Vorrichtung |
| 107 | Signalleser |
| 108 | Vektordaten-Generator |
| 109 | Vektordaten-Speicher |
| 110 | Vektordaten-Auswerter |
| 111 | Vektordaten-Synchronisierer |
| 112 | Vektordaten-Anzeige |
| 113 | Server |
| 114 | Vektordaten-Speicher |
| 115 | Vektordaten-Analysator |
| 116 | Vektordaten-Auswerte-Speicher |
| A | anteriore Ableitung |
| D | dorsale Ableitung |
| E1 | erste Ableitungspunkt |
| E2 | zweiter Ableitungspunkt |
| E3 | dritter Ableitungspunkt |
| E4 | vierte Ableitungspunkt |
| f | spezifische Lernwerte (für das neuronale Netz 26) |
| H | horizontale Ableitung |
| I | inferiore Ableitung |
| M | Menge an Patienten (mit bekanntem Befund) |
| $PC_1$ | erste Hauptachse |
| $PC_2$ | zweite Hauptachse |

S1-S4      Sensoren, die den Ableitungspunkten E1-E4 zugeordnet sind
V      vertikale Ableitung
$\alpha$      Nicht-rechtwinklige Winkel des ersten Dreiecks 21 (E1-E3-E4)
$\beta$      Nicht-rechtwinklige Winkel des zweiten Dreiecks 22 (E1-E2-E4)
$\varepsilon, \eta$      Anpassungswerte für die Winkelkorrektur, für die Winkel $\alpha$ und $\beta$

**Patentansprüche**

1. Herzüberwachungsverfahren, bei dem auf Grundlage von am Herzen (12) eines Patienten aufgenommenen EKG-Signalen ein Raumvektor (24) anhand der Vektorkardiographie bestimmt wird, wobei dieser Raumvektor (24) den zeitlichen Verlauf des Summenvektors des elektrischen Feldes des Herzens (12) abbildet und eine der Feldrichtung entsprechende Richtung und eine dem Potential entsprechende Länge aufweist,
**dadurch gekennzeichnet,**

   **dass** ein Quotient aus den Flächen, die von einer Länge des Raumvektors (24) (= Radiusvektor) als Funktion der Zeit jeweils während der R-Welle und während der T-Welle überstrichen werden, gebildet wird, wobei anschließend dieser Quotient einer weiteren Auswertung unter Berücksichtigung von zumindest einem vorbestimmten Grenzwert zugeführt wird, um das Vorliegen einer koronaren Herzkrankheit (KHK) und/oder einer Herzrhythmusstörung für einen Patienten zu überprüfen,
   wobei für das untersuchte Herz (12) eine koronare Herzkrankheit (KHK) erkannt wird, falls der aus den von dem Raumvektor (24) jeweils während der R-Welle und während der T-Welle überstrichenen Flächen gebildete Quotient

$$\frac{\text{Fläche (R-Welle)}}{\text{Fläche (T-Welle)}} = a$$

   ausserhalb eines durch einen unteren Grenzwert $a_{0,KHK}$ und einen oberen Grenzwert $a_{1,KHK}$ gebildeten Intervalls liegt, und
   **dass** die Grenzwerte $a_{0,KHK}$ und $a_{1,KHK}$ in Abhängigkeit von einem Trainingsset bestimmt werden,
   wobei die unteren und oberen Grenzwerte $a_0$ und $a_1$ jeweils mit Hilfe eines Trainingssets von Probanden-Daten bestimmt werden, mit den Schritten:

       (i) Berechnung des Quotienten Fläche (R-Welle) / Fläche (T-Welle) für alle Probanden, um damit Ratio-Zeitreihen zu erhalten,
       (ii) Berechnung eines Mittelwertes ($\mu$) und einer zugehörigen Standardabweichung ($\sigma$) aus den Ratio-Zeitreihen von Schritt (i),
       (iii) Bestimmung des unteren Grenzwertes $a_0$ unter Berücksichtung des Mittelwertes ($\mu$) und der Standardabweichung ($\sigma$) von Schritt (ii), durch die Beziehung:

$$a_0 = \text{Mittelwert } (\mu) - \text{Standardabweichung } (\sigma),$$

       und/oder
       Bestimmung des oberen Grenzwertes $a_1$ unter Berücksichtung des Mittelwertes ($\mu$) und der Standardabweichung ($\sigma$) von Schritt (ii), durch die Beziehung:

$$a_1 = \text{Mittelwert } (\mu) + \text{Standardabweichung } (\sigma),$$

       wobei in Schritt (ii) der Mittelwert und die zugehörige Standardabweichung nur für Ratio-Zeitreihen von gesunden Patienten ("- -") berechnet werden.

2. Verfahren nach Anspruch 1, **dadurch gekennzeichnet, dass** für das untersuchte Herz (12) eine Herzrhythmusstörung (HRS) erkannt wird, falls der aus den von dem Raumvektor (24) jeweils während der R-Welle und während der T-Welle überstrichenen Flächen gebildete Quotient die Bedingung

$$\frac{\text{Fläche (R-Welle)}}{\text{Fläche (T-Welle)}} < a_{0,HRS}$$

oder die Bedingung

$$\frac{\text{Fläche (R-Welle)}}{\text{Fläche (T-Welle)}} > a_{1,HRS}$$

erfüllt.

3. Verfahren nach Anspruch 2, **dadurch gekennzeichnet, dass** der untere Grenzwert $a_{0,HRS}$ und/oder der obere Grenzwert $a_{1,HRS}$ in Abhängigkeit von einem Trainingsset bestimmt werden.

4. Verfahren nach einem der vorhergehenden Ansprüche, **dadurch gekennzeichnet, dass** der Raumvektor (24) nach einem Verfahren ermittelt wird, das zur Ermittlung und Darstellung eines auf das Herz (12) bezogenen Raumvektors (24) dient, der den Vektor des elektrischen Feldes darstellt, das durch die Aktivität des Herzens (12) gebildet wird, wobei Messwerte des Herzens (12) am Körper (14) an einem ersten Ableitungspunkt (E1), an einem zweiten Ableitungspunkt (E2), an einem dritten Ableitungspunkt (E3) und an einem vierten Ableitungspunkt (E4) erfasst werden, wobei Potentialdifferenzen in Form einer anterioren Ableitung (A) zwischen dem ersten Ableitungspunkt (E1) und dem vierten Ableitungspunkt (E4), einer dorsalen Ableitung (D) zwischen dem zweiten Ableitungspunkt (E2) und dem vierten Ableitungspunkt (E4), einer horizontalen Ableitung (H) zwischen dem dritten Ableitungspunkt (E3) und dem vierten Ableitungspunkt (E4), einer vertikalen Ableitung (V) zwischen dem ersten Ableitungspunkt (E1) und dem dritten Ableitungspunkt (E3), und einer inferioren Ableitung (I) zwischen dem ersten Ableitungspunkt (E1) und dem zweiten Ableitungspunkt (E2) gemessen werden, wobei ein orthogonales System mit den Beziehungen:

$$x = D \cos 45° - I$$

$$y = D \sin 45° + A$$

$$z = (V - H) \sin 45°$$

gebildet wird und die Messwerte und der daraus ermittelte Raumvektor (24) in diesem orthogonalen System (x, y, z) abgebildet werden,
**gekennzeichnet durch** folgende Schritte:

(a) Durchführung einer Messung an einem Patienten unter Verwendung der ersten bis vierten Ableitungspunkte (E1-E4) am Körper (14) des Patienten, um damit für diesen Patienten ein Kardiogramm zu gewinnen,
(b) Extrahieren der Amplituden der R-Welle aus dem Kardiogramm von Schritt (a) für jeden Herzschlag in x- , y- und z-Richtung,
(c) Ermitteln von Mittelwerten $\mu_x$, $\mu_y$, $\mu_z$ und Standard-Abweichungen $\sigma_x$, $\sigma_y$, $\sigma_z$ der jeweils in Millivolt erfassten Amplituden aus dem Kardiogramm gemäß Schritt (b), wobei mit diesen Mittelwerten und Standard-Abweichungen dann ein Berechnungsvektor (26) gebildet wird,
(d) Bilden einer Koeffizientenmatrix (28), die auf Grundlage einer Hauptachsentransformation für Messungen an Vergleichs-Patienten mit unterschiedlichen Anlageschemata gewonnen worden ist,
(e) Multiplizieren des Berechnungsvektors (26) von Schritt (c) mit der Koeffizientenmatrix (28) von Schritt (d), zur Bildung eines Ergebnisvektors (30) mit insgesamt sechs Hauptachsen ($PC_1$ - $PC_6$), wobei es sich bei der Koeffizientenmatrix (28), mit welcher in Schritt (e) der Berechnungsvektor (26) zur Bildung des Ergebnisvektors (30) multipliziert wird, um eine 6x6-Koeffizientenmatrix handelt,
(f) Extrahieren der ersten Hauptachse ($PC_1$) und der zweiten Hauptachse ($PC_2$) aus dem Ergebnisvektor (30) von Schritt (e), zur Bildung eines Referenzpunktes ($PC_1$, $PC_2$) im Raum der ersten und zweiten Hauptachse,

(g) Ermitteln eines euklidischen Abstands des Referenzpunktes ($PC_1$, $PC_2$) von einem vorbestimmten Zielpunkt ($PC_1^{fit}$, $PC_2^{fit}$), der einer korrekten Position der vier Ableitungspunkte (E1-E4) am menschlichen Körper (14) entspricht, und

(h) falls der Abstand des Referenzpunktes ($PC_1$, $PC_2$) von dem vorbestimmten Zielpunkt ($PC_1^{fit}$, $PC_2^{fit}$) größer als ein vorbestimmter Maximalwert ist: Durchführen einer Winkelkorrektur für ein aus dem ersten Ableitungspunkt (E1), dem dritten Ableitungspunkt (E3) und dem vierten Ableitungspunkt (E4) gebildetes erstes Dreieck (31) und für ein aus dem ersten Ableitungspunkt (E1), dem zweiten Ableitungspunkt (E2) und dem vierten Ableitungspunkt (E4) gebildetes zweites Dreieck (32), so dass damit der euklidische Abstand zwischen dem Referenzpunkt ($PC_1$, $PC_2$) und dem vorbestimmten Zielpunkt ($PC_1^{fit}$, $PC_2^{fit}$) durch Anpassung des orthogonalen Systems (x, y, z) an die geänderte Geometrie minimiert wird, wobei in Schritt (h) durch die Winkelkorrektur Anpassungswerte ($\varepsilon$, $\eta$) ermittelt werden, mit denen die nicht-rechtwinkligen Winkel ($\alpha$) des ersten Dreiecks (31) und die nicht-rechtwinkligen Winkel ($\beta$) des zweiten Dreiecks (32) korrigiert werden, so dass dadurch zumindest eine nicht korrekte Position des ersten Ableitungspunkts (E1) am menschlichen Körper (14) kompensiert wird.

5. Verfahren nach Anspruch 4, **dadurch gekennzeichnet, dass** vor Durchführung des Schritts (h) der vorbestimmte Zielpunkt ($PC_1$, $PC_2$) aus einem Zielgebiet (34) ausgewählt wird, das auf Grundlage von Standardabweichungen einer Mehrzahl von überprüften korrekten Messungen des Zielpunktes am menschlichen Körper (14) gebildet wird.

6. Verfahren nach Anspruch 4 oder 5, **dadurch gekennzeichnet, dass** in Schritt (h) für die Winkelkorrektur die Anpassungswerte ($\varepsilon$, $\eta$) bestimmt werden durch Minimierung des euklidischen Abstands zwischen Referenz- und Zielwerten.

**Claims**

1. A heart monitoring method in which ECG signals are recorded at the heart (12) of a patient and on the basis of which a space vector (24) is determined using vectorcardiography, wherein this space vector (24) represents the course of the sum vector of the electrical field of the heart (12) and has a direction corresponding to the field direction and a length corresponding to the potential,
**characterized in**

**that** a quotient is formed from the areas covered by a length of the space vector (24) (= radius vector) as a function of time during the R-wave and during the T-wave, respectively, wherein this quotient is then subjected to further evaluation taking into account at least one predetermined limit value, in order to verify the presence of coronary heart disease (KHK) and / or cardiac arrhythmia for a patient,
wherein coronary heart disease (KHK) is detected for the screened heart (12) if the quotient formed from the areas covered by the space vector (24) during the R-wave and during the T-wave, respectively

$$\frac{\text{Area (R-wave)}}{\text{Area (T-wave)}} = a$$

is located outside a range defined by a lower limit value $a_{0,KHK}$ and an upper limit value $a_{1,KHK}$, and
**that** the limit values $a_{0,KHK}$ and $a_{1,KHK}$ are determined as a function of a training set,
wherein the lower and upper limit values $a_0$ and $a_1$ are respectively determined by a training set of test person data, comprising the steps:

(i) calculating the quotient area (R-wave) / area (T-wave) for all test persons to obtain time series ratios;
(ii) calculating a mean value ($\mu$) and an associated standard deviation ($\sigma$) from the time series ratios of step (i); and
(iii) determining the lower limit $a_0$ taking into account the mean value ($\mu$) and the standard deviation ($\sigma$) of step (ii), by the relationship:

$$a_0 = \text{mean value } (\mu) - \text{standard deviation } (\sigma),$$

and / or

determining the upper limit $a_1$ taking into account the mean value ($\mu$) and the standard deviation ($\sigma$) of step (ii), by the relationship:

$$a_1 = \text{mean value } (\mu) + \text{standard deviation } (\sigma),$$

wherein in step (ii) the mean value and the associated standard deviation are calculated only for time series ratios of healthy patients ("- -").

2. The method according to claim 1, wherein a cardiac arrhythmia (HRS) is detected for the screened heart (12) if the quotient formed by the areas covered by the space vector (24) during the R-wave and during the T-wave, respectively, satisfies the condition of

$$\frac{\text{Area (R-wave)}}{\text{Area (T-wave)}} < a_{0,HRS}$$

or the condition of

$$\frac{\text{Area (R-wave)}}{\text{Area (T-wave)}} > a_{1,HRS}$$

3. The method according to claim 2, wherein the lower limit value $a_{0,KHK}$ and/or the upper limit value $a_{1,KHK}$ are determined as a function of a training set.

4. The method according to any of the preceding claims, wherein the space vector (24) is determined according to a method which serves for determining and displaying a space vector (24) related to the heart (12), which represents the vector of the electrical field formed by the activity of the heart (12), wherein measured values of the heart (12) are recorded on the body (14) at a first lead point (E1), at a second lead point (E2), at a third lead point (E3) and at a fourth lead point (E4), wherein potential differences are measured in the form of an anterior lead (A) between the first lead point (E1) and the fourth lead point (E4), a dorsal lead (D) between the second lead point (E2) and the fourth lead point (E4), a horizontal lead (H) between the third lead point (E3) and the fourth lead point (E4), a vertical lead (V) between the first lead point (E1) and the third lead point (E3), and an inferior lead (I) between the first lead point (E1) and the second lead point (E2), wherein an orthogonal system is formed with the relationships:

$$x = D \cos 45° - I$$

$$y = D \sin 45° + A$$

$$z = (V - H) \sin 45°$$

and the measured values and the space vector (24) determined therefrom are mapped in this orthogonal system (x, y, z),
**characterized by** the following steps:

(a) performing a measurement on a patient using the first to fourth lead points (E1-E4) on the body (14) of the patient in order to obtain a cardiogram for this patient,
(b) extracting the amplitudes of the R-wave from the cardiogram of step (a) for each heartbeat in the x, y and z direction,
(c) determining mean values $\mu_x$, $\mu_y$, $\mu_z$ and standard deviations $\sigma_x$, $\sigma_y$, $\sigma_z$ of the respective amplitudes recorded in millivolts from the cardiogram in step (b), wherein these mean values and standard deviations then form a calculation vector (26),
(d) forming a coefficient matrix (28) which is obtained based on a Principal Component Analysis by using different formats for measurements on reference patients,

(e) multiplying the calculation vector (26) of step (c) by the coefficient matrix of step (d) to form a resulting vector (30) with a total of six main axes ($PC_1$ - $PC_6$), wherein the coefficient matrix (28), with which in step (e) the calculation vector (26) is multiplied to form the resulting vector (30), is a 6x6 coefficient matrix,

(f) extracting the first main axis ($PC_1$) and the second main axis ($PC_2$) from the resulting vector (30) of step (e) to form a reference point ($PC_1$, $PC_2$) in the space of the first and second main axis,

(g) determining an Euclidean distance of the reference point ($PC_1$, $PC_2$) from a predetermined target point ($PC_1^{fit}$, $PC_2^{fit}$) which corresponds to a correct position of the four lead points (E1-E4) on the human body (14), and

(h) if the distance of the reference point ($PC_1$, $PC_2$) from the predetermined target point ($PC_1^{fit}$, $PC_2^{fit}$) is greater than a predetermined maximum value: performing an angular correction for a first triangle (31) formed by the first lead point (E1), the third lead point (E3) and the fourth lead point (E4), and for a second triangle (32) formed by the first lead point (E1), the second lead point (E2) and the fourth lead point (E4) so that thereby the Euclidean distance between the reference point ($PC_1$, $PC_2$) and the predetermined target point ($PC_1^{fit}$, $PC_2^{fit}$) is minimized by adapting the orthogonal system (x, y, z) to the changed geometry, wherein in step (h) the angular correction determines adjustment values ($\varepsilon$, $\eta$) with which the non-orthogonal angles ($\alpha$) of the first triangle (31) and the non-orthogonal angles ($\beta$) of the second triangle (32) are corrected, so that at least an incorrect position of the first lead point (E1) on the human body (14) is compensated.

5. The method according to claim 4, wherein prior to performing step (h), the predetermined target point ($PC_1$, $PC_2$) is selected from a target area (34), which is formed on the basis of standard deviations of a plurality of verified correct measurements of the target point on the human body (14).

6. The method according to claim 4 or 5, wherein in step (h) for the angular correction the adjusement values are determined by minimizing the Euclidean distance between reference and target values.

**Revendications**

1. Procédé de surveillance cardiaque, dans lequel, sur la base de signaux ECG enregistrés sur le coeur (12) d'un patient, un vecteur spatial (24) est déterminé à l'aide de la cardiographie vectorielle, ce vecteur spatial (24) reproduisant l'évolution dans le temps du vecteur somme du champ électrique du coeur (12) et présentant une direction correspondant à la direction du champ et une longueur correspondant au potentiel,
**caractérisé en ce que**

qu'un quotient est formé à partir des surfaces qui sont balayées par une longueur du vecteur spatial (24) (= vecteur de rayon) en fonction du temps respectivement pendant l'onde R et pendant l'onde **T,** ce quotient étant ensuite amené à une autre évaluation en tenant compte d'au moins une valeur limite prédéterminée, afin de vérifier la présence d'une maladie coronarienne (MCC) et/ou d'un trouble du rythme cardiaque pour un patient, une maladie coronarienne étant détectée pour le coeur (12) examiné si le quotient

$$\frac{\text{Surface (arbre R)}}{\text{Surface (onde T)}} = a$$

se situe en dehors d'un intervalle formé par une valeur limite inférieure $a_{0,KHK}$ et une valeur limite supérieure $a_{1,KHK}$ , et
que les valeurs limites $a_{0,KHK}$ et $a_{1,KHK}$ sont déterminées en fonction d'un ensemble d'entraînement,
les valeurs limites inférieure et supérieure $a_0$ et $a_1$ étant respectivement déterminées à l'aide d'un ensemble d'apprentissage de données de sujets, avec les étapes:

(i) calculer du quotient surface (onde R)/surface (onde T) pour tous les sujets afin d'obtenir des séries temporelles de ratios,
(ii) calculer une moyenne ($\mu$) et un écart-type associé ($\sigma$) à partir des séries temporelles de ratios de l'étape (i),
(iii) déterminer la valeur limite inférieure $a_0$ en tenant compte de la valeur moyenne ($\mu$) et de l'écart type ($\sigma$) de l'étape (ii), par la relation:

$$a_0 = \text{moyenne } (\mu) - \text{écart-type } (\sigma),$$

et/ou

déterminer la valeur limite supérieure $a_1$ en tenant compte de la moyenne $(\mu)$ et de l'écart type $(\sigma)$ de l'étape (ii), par la relation :

$$a_1 = \text{moyenne } (\mu) + \text{écart-type } (\sigma),$$

où, à l'étape (ii), la moyenne et l'écart-type correspondant sont calculés uniquement pour des séries temporelles de ratios de patients sains ("- -").

2. Procédé selon la revendication 1, **caractérisé en ce qu'**un trouble du rythme cardiaque (HRS) est détecté pour le coeur (12) examiné si le quotient formé par les surfaces balayées par le vecteur spatial (24) respectivement pendant l'onde R et pendant l'onde T satisfait à la condition

$$\frac{\text{Surface (arbre R)}}{\text{Surface (onde T)}} < a_{0,HRS}$$

ou à la condition

$$\frac{\text{Surface (arbre R)}}{\text{Surface (onde T)}} > a_{1,HRS}$$

3. Procédé selon la revendication 2, **caractérisé en ce que** la valeur limite inférieure $a_{0,HRS}$ et/ou la valeur limite supérieure $a_{1,HRS}$ sont déterminées en fonction d'un ensemble d'entraînement.

4. Procédé selon l'une des revendications précédentes, **caractérisé en ce que** le vecteur spatial (24) est déterminé selon un procédé qui sert à déterminer et à représenter un vecteur spatial (24) relatif au cœur (12), qui représente le vecteur du champ électrique formé par l'activité du cœur (12), des valeurs de mesure du cœur (12) étant enregistrées sur le corps (14) en un premier point de dérivation (E1), en un deuxième point de dérivation (E2), en un troisième point de dérivation (E3) et en un quatrième point de dérivation (E4), où des différences de potentiel sont mesurées sous la forme d'une dérivée antérieure (A) entre le premier point de dérivation (E1) et le quatrième point de dérivation (E4), d'une dérivée dorsale (D) entre le deuxième point de dérivation (E2) et le quatrième point de dérivation (E4), d'une dérivée horizontale (H) entre le troisième point de dérivation (E3) et le quatrième point de dérivation (E4), d'une dérivée verticale (V) entre le premier point de dérivation (E1) et le troisième point de dérivation (E3), et d'une dérivée inférieure (I) entre le premier point de dérivation (E1) et le deuxième point de dérivation (E2), un système orthogonal ayant les relations:

$$x = D \cos 45° - I$$

$$y = D \sin 45° + A$$

$$z = (V - H) \sin 45$$

et les valeurs de mesure et le vecteur spatial (24) qui en résulte sont représentés dans ce système orthogonal (x, y, z),
**caractérisé par** les étapes suivantes :

(a) Réalisation d'une mesure sur un patient en utilisant les premier à quatrième points de dérivation (E1-E4) sur le corps (14) du patient, afin d'obtenir ainsi un cardiogramme pour ce patient,

(b) Extraction les amplitudes de l'onde R du cardiogramme de l'étape (a) pour chaque battement de cœur dans les directions x, y et z,

(c) Détermination des valeurs moyennes $\mu_x$, $\mu_y$, $\mu_z$ et des écarts types $\sigma_x$, $\sigma_y$, $\sigma_z$ des amplitudes respectivement saisies en millivolts à partir du cardiogramme selon l'étape (b), un vecteur de calcul (26) étant ensuite formé avec ces valeurs moyennes et ces écarts types,

(d) Formation d'une matrice de coefficients (28) obtenue sur la base d'une transformation de l'axe principal pour des mesures sur des patients de comparaison avec différents schémas d'installation,

(e) multiplier le vecteur de calcul (26) de l'étape (c) par la matrice de coefficients (28) de l'étape (d), pour former un vecteur de résultat (30) ayant un total de six axes principaux ($PC_1$ - $PC_6$), la matrice de coefficients (28) par laquelle le vecteur de calcul (26) est multiplié à l'étape (e) pour former le vecteur de résultat (30) étant une matrice de coefficients 6x6,

(f) Extraction du premier axe principal ($PC_1$) et du deuxième axe principal ($PC_2$) à partir du vecteur résultat (30) de l'étape (e), pour former un point de référence ($PC_1$, $PC_2$) dans l'espace du premier et du deuxième axe principal,

(g) Détermination d'une distance euclidienne du point de référence ($PC_1$, $PC_2$) par rapport à un point cible prédéterminé ($PC_1^{fit}$, $PC_2^{fit}$) correspondant à une position correcte des quatre points de dérivation (E1-E4) sur le corps humain (14), et

(h) si la distance entre le point de référence ($PC_1$, $PC_2$) et le point cible prédéterminé ($PC_1^{fit}$, $PC_2^{fit}$) est supérieure à une valeur maximale prédéterminée: effectuer une correction angulaire pour un premier triangle (31) formé par le premier point de dérivation (E1), le troisième point de dérivation (E3) et le quatrième point de dérivation (E4) et pour un deuxième triangle (32) formé par le premier point de dérivation (E1), le deuxième point de dérivation (E2) et le quatrième point de dérivation (E4), de sorte que la distance euclidienne entre le point de référence ($PC_1$, $PC_2$) et le point cible prédéterminé ($PC_1^{fit}$, $PC_2^{fit}$) est minimisée par adaption du système orthogonal (x, y, z) à la géométrie modifiée, des valeurs d'adaptation ($\varepsilon$, $\eta$) étant déterminées à l'étape (h) par la correction angulaire, avec lesquelles les angles non rectangulaires ($\alpha$) du premier triangle (31) et les angles non rectangulaires ($\beta$) du deuxième triangle (32) sont corrigés, de sorte qu'au moins une position non correcte du premier point de dérivation (E1) sur le corps humain (14) est ainsi compensée.

5. Procédé selon la revendication 4, **caractérisé en ce que**, avant l'exécution de l'étape (h), le point cible prédéterminé ($PC_1$, $PC_2$) est sélectionné à partir d'une zone cible (34) formée sur la base des écarts types d'une pluralité de mesures correctes vérifiées du point cible sur le corps humain (14).

6. Procédé selon la revendication 4 ou 5, **caractérisé en ce que**, à l'étape (h), pour la correction angulaire, les valeurs d'ajustement ($\varepsilon$, $\eta$) sont déterminées en minimisant l'écart euclidien entre les valeurs de référence et les valeurs cibles.

10    16              18              20

S1    S2    S3    S4

Fig. 1

E4 ——————————— ○          ○ ——————————— E3
                              ◌ ————————————— E2
14 ———————————             ○
                                    E1

**Fig. 2a**

E3 ——————————— ◌          ◌ ——————————— E4
E2 ———————————    ○
E1 ———————————    ◌
14 ———————————

**Fig. 2b**

**Fig. 3a**

**Fig. 3b**

**Fig. 4**

**Fig. 5a**

**Fig. 5b**

**Fig. 6**

**Fig. 7a**

**Fig. 7b**

(i)  (ii)  (iii)  (iv)

**Fig. 8**

(i)  (ii)  (iii)  (iv)

(v)  (vi)  (vii)  (viii)  (ix)  (x)  (xi)  (xii)

f

**Fig. 9**

EP 4 239 648 B1

**Fig. 10a**

**Fig. 10b**

30

1. Mag-Difference-QRS_begin-R
2. Mag-Difference-QRS_begin-QRS_end
3. Mag-Difference-QRS_begin-T_begin
4. Mag-Difference-QRS_begin-T
5. Mag-Difference-QRS_begin-T_end
6. Mag-Difference-R-QRS_end
7. Mag-Difference-R-T_begin
8. Mag-Difference-R-T
9. Mag-Difference-R-T_end
10. Mag-Difference-QRS_end-T_begin
11. Mag-Difference-QRS_end-T
12. Mag-Difference-QRS_end-T_end
13. Mag-Difference-T_begin-T
14. Mag-Difference-T_begin-T_end
15. Mag-Difference-T-T_end
16. Azi-Difference-QRS_begin-R
17. Azi-Difference-QRS_begin-QRS_end
18. Azi-Difference-QRS_begin-T_begin
19. Azi-Difference-QRS_begin-T
20. Azi-Difference-QRS_begin-T_end
21. Azi-Difference-R-QRS_end
22. Azi-Difference-R-T_begin
23. Azi-Difference-R-T
24. Azi-Difference-R-T_end
25. Azi-Difference-QRS_end-T_begin
26. Azi-Difference-QRS_end-T
27. Azi-Difference-QRS_end-T_end
28. Azi-Difference-T_begin-T
29. Azi-Difference-T_begin-T_end
30. Azi-Difference-T-T_end
31. Ele-Difference-QRS_begin-R
32. Ele-Difference-QRS_begin-QRS_end
33. Ele-Difference-QRS_begin-T_begin
34. Ele-Difference-QRS_begin-T
35. Ele-Difference-QRS_begin-T_end
36. Ele-Difference-R-QRS_end
37. Ele-Difference-R-T_begin
38. Ele-Difference-R-T
39. Ele-Difference-R-T_end
40. Ele-Difference-QRS_end-T_begin
41. Ele-Difference-QRS_end-T
42. Ele-Difference-QRS_end-T_end
43. Ele-Difference-T_begin-T
44. Ele-Difference-T_begin-T_end
45. Ele-Difference-T-T_end
46. Mag-Triangle-QRS_begin-R-QRS_end
47. Mag-Triangle-QRS_begin-R-T_begin
48. Mag-Triangle-QRS_begin-R-T
49. Mag-Triangle-QRS_begin-R-T_end
50. Mag-Triangle-QRS_begin-QRS_end-T_begin
51. Mag-Triangle-QRS_begin-QRS_end-T
52. Mag-Triangle-QRS_begin-QRS_end-T_end
53. Mag-Triangle-QRS_begin-T_begin-T
54. Mag-Triangle-QRS_begin-T_begin-T_end
55. Mag-Triangle-QRS_begin-T-T_end
56. Mag-Triangle-R-QRS_end-T_begin
57. Mag-Triangle-R-QRS_end-T
58. Mag-Triangle-R-QRS_end-T_end
59. Mag-Triangle-R-T_begin-T
60. Mag-Triangle-R-T_begin-T_end
61. Mag-Triangle-R-T-T_end
62. Mag-Triangle-QRS_end-T_begin-T
63. Mag-Triangle-QRS_end-T_begin-T_end
64. Mag-Triangle-QRS_end-T-T_end
65. Mag-Triangle-T_begin-T-T_end
66. Cartesian-TimeDiff-QRS_beginX-QRS_beginY
67. Cartesian-TimeDiff-QRS_beginX-QRS_beginZ
68. Cartesian-TimeDiff-QRS_beginX-RX
69. Cartesian-TimeDiff-QRS_beginX-RY
70. Cartesian-TimeDiff-QRS_beginX-RZ
71. Cartesian-TimeDiff-QRS_beginX-QRS_endX
72. Cartesian-TimeDiff-QRS_beginX-QRS_endY
73. Cartesian-TimeDiff-QRS_beginX-QRS_endZ
74. Cartesian-TimeDiff-QRS_beginX-T_beginX
75. Cartesian-TimeDiff-QRS_beginX-T_beginY
76. Cartesian-TimeDiff-QRS_beginX-T_beginZ
77. Cartesian-TimeDiff-QRS_beginX-TX
78. Cartesian-TimeDiff-QRS_beginX-TY
79. Cartesian-TimeDiff-QRS_beginX-TZ
80. Cartesian-TimeDiff-QRS_beginX-T_endX
81. Cartesian-TimeDiff-QRS_beginX-T_endY
82. Cartesian-TimeDiff-QRS_beginX-T_endZ
83. Cartesian-TimeDiff-QRS_beginY-QRS_beginZ
84. Cartesian-TimeDiff-QRS_beginY-RX
85. Cartesian-TimeDiff-QRS_beginY-RY
86. Cartesian-TimeDiff-QRS_beginY-RZ

**Fig. 11a**

87. Cartesian-TimeDiff-QRS_beginY-QRS_endX

88. Cartesian-TimeDiff-QRS_beginY-QRS_endY

89. Cartesian-TimeDiff-QRS_beginY-QRS_endZ

90. Cartesian-TimeDiff-QRS_beginY-T_beginX

91. Cartesian-TimeDiff-QRS_beginY-T_beginY

92. Cartesian-TimeDiff-QRS_beginY-T_beginZ

93. Cartesian-TimeDiff-QRS_beginY-TX

94. Cartesian-TimeDiff-QRS_beginY-TY

95. Cartesian-TimeDiff-QRS_beginY-TZ

96. Cartesian-TimeDiff-QRS_beginY-T_endX

97. Cartesian-TimeDiff-QRS_beginY-T_endY

98. Cartesian-TimeDiff-QRS_beginY-T_endZ

99. Cartesian-TimeDiff-QRS_beginZ-RX

100. Cartesian-TimeDiff-QRS_beginZ-RY

101. Cartesian-TimeDiff-QRS_beginZ-RZ

102. Cartesian-TimeDiff-QRS_beginZ-QRS_endX

103. Cartesian-TimeDiff-QRS_beginZ-QRS_endY

104. Cartesian-TimeDiff-QRS_beginZ-QRS_endZ

105. Cartesian-TimeDiff-QRS_beginZ-T_beginX

106. Cartesian-TimeDiff-QRS_beginZ-T_beginY

107. Cartesian-TimeDiff-QRS_beginZ-T_beginZ

108. Cartesian-TimeDiff-QRS_beginZ-TX

109. Cartesian-TimeDiff-QRS_beginZ-TY

110. Cartesian-TimeDiff-QRS_beginZ-TZ

111. Cartesian-TimeDiff-QRS_beginZ-T_endX

112. Cartesian-TimeDiff-QRS_beginZ-T_endY

113. Cartesian-TimeDiff-QRS_beginZ-T_endZ

114. Cartesian-TimeDiff-RX-RY

115. Cartesian-TimeDiff-RX-RZ

116. Cartesian-TimeDiff-RX-QRS_endX

117. Cartesian-TimeDiff-RX-QRS_endY

118. Cartesian-TimeDiff-RX-QRS_endZ

119. Cartesian-TimeDiff-RX-T_beginX

120. Cartesian-TimeDiff-RX-T_beginY

121. Cartesian-TimeDiff-RX-T_beginZ

122. Cartesian-TimeDiff-RX-TX

123. Cartesian-TimeDiff-RX-TY

124. Cartesian-TimeDiff-RX-TZ

125. Cartesian-TimeDiff-RX-T_endX

126. Cartesian-TimeDiff-RX-T_endY

127. Cartesian-TimeDiff-RX-T_endZ

128. Cartesian-TimeDiff-RY-RZ

129. Cartesian-TimeDiff-RY-QRS_endX

130. Cartesian-TimeDiff-RY-QRS_endY

131. Cartesian-TimeDiff-RY-QRS_endZ

132. Cartesian-TimeDiff-RY-T_beginX

133. Cartesian-TimeDiff-RY-T_beginY

134. Cartesian-TimeDiff-RY-T_beginZ

135. Cartesian-TimeDiff-RY-TX

136. Cartesian-TimeDiff-RY-TY

137. Cartesian-TimeDiff-RY-TZ

138. Cartesian-TimeDiff-RY-T_endX

139. Cartesian-TimeDiff-RY-T_endY

140. Cartesian-TimeDiff-RY-T_endZ

141. Cartesian-TimeDiff-RZ-QRS_endX

142. Cartesian-TimeDiff-RZ-QRS_endY

143. Cartesian-TimeDiff-RZ-QRS_endZ

144. Cartesian-TimeDiff-RZ-T_beginX

145. Cartesian-TimeDiff-RZ-T_beginY

146. Cartesian-TimeDiff-RZ-T_beginZ

147. Cartesian-TimeDiff-RZ-TX

148. Cartesian-TimeDiff-RZ-TY

149. Cartesian-TimeDiff-RZ-TZ

150. Cartesian-TimeDiff-RZ-T_endX

151. Cartesian-TimeDiff-RZ-T_endY

152. Cartesian-TimeDiff-RZ-T_endZ

153. Cartesian-TimeDiff-QRS_endX-QRS_endY

154. Cartesian-TimeDiff-QRS_endX-QRS_endZ

155. Cartesian-TimeDiff-QRS_endX-T_beginX

156. Cartesian-TimeDiff-QRS_endX-T_beginY

157. Cartesian-TimeDiff-QRS_endX-T_beginZ

158. Cartesian-TimeDiff-QRS_endX-TX

159. Cartesian-TimeDiff-QRS_endX-TY

160. Cartesian-TimeDiff-QRS_endX-TZ

161. Cartesian-TimeDiff-QRS_endX-T_endX

162. Cartesian-TimeDiff-QRS_endX-T_endY

163. Cartesian-TimeDiff-QRS_endX-T_endZ

164. Cartesian-TimeDiff-QRS_endY-QRS_endZ

165. Cartesian-TimeDiff-QRS_endY-T_beginX

166. Cartesian-TimeDiff-QRS_endY-T_beginY

167. Cartesian-TimeDiff-QRS_endY-T_beginZ

168. Cartesian-TimeDiff-QRS_endY-TX

169. Cartesian-TimeDiff-QRS_endY-TY

170. Cartesian-TimeDiff-QRS_endY-TZ

171. Cartesian-TimeDiff-QRS_endY-T_endX

172. Cartesian-TimeDiff-QRS_endY-T_endY

## Fig. 11b

173. Cartesian-TimeDiff-QRS_endY-T_endZ

174. Cartesian-TimeDiff-QRS_endZ-T_beginX

175. Cartesian-TimeDiff-QRS_endZ-T_beginY

176. Cartesian-TimeDiff-QRS_endZ-T_beginZ

177. Cartesian-TimeDiff-QRS_endZ-TX

178. Cartesian-TimeDiff-QRS_endZ-TY

179. Cartesian-TimeDiff-QRS_endZ-TZ

180. Cartesian-TimeDiff-QRS_endZ-T_endX

181. Cartesian-TimeDiff-QRS_endZ-T_endY

182. Cartesian-TimeDiff-QRS_endZ-T_endZ

183. Cartesian-TimeDiff-T_beginX-T_beginY

184. Cartesian-TimeDiff-T_beginX-T_beginZ

185. Cartesian-TimeDiff-T_beginX-TX

186. Cartesian-TimeDiff-T_beginX-TY

187. Cartesian-TimeDiff-T_beginX-TZ

188. Cartesian-TimeDiff-T_beginX-T_endX

189. Cartesian-TimeDiff-T_beginX-T_endY

190. Cartesian-TimeDiff-T_beginX-T_endZ

191. Cartesian-TimeDiff-T_beginY-T_beginZ

192. Cartesian-TimeDiff-T_beginY-TX

193. Cartesian-TimeDiff-T_beginY-TY

194. Cartesian-TimeDiff-T_beginY-TZ

195. Cartesian-TimeDiff-T_beginY-T_endX

196. Cartesian-TimeDiff-T_beginY-T_endY

197. Cartesian-TimeDiff-T_beginY-T_endZ

198. Cartesian-TimeDiff-T_beginZ-TX

199. Cartesian-TimeDiff-T_beginZ-TY

200. Cartesian-TimeDiff-T_beginZ-TZ

201. Cartesian-TimeDiff-T_beginZ-T_endX

202. Cartesian-TimeDiff-T_beginZ-T_endY

203. Cartesian-TimeDiff-T_beginZ-T_endZ

204. Cartesian-TimeDiff-TX-TY

205. Cartesian-TimeDiff-TX-TZ

206. Cartesian-TimeDiff-TX-T_endX

207. Cartesian-TimeDiff-TX-T_endY

208. Cartesian-TimeDiff-TX-T_endZ

209. Cartesian-TimeDiff-TY-TZ

210. Cartesian-TimeDiff-TY-T_endX

211. Cartesian-TimeDiff-TY-T_endY

212. Cartesian-TimeDiff-TY-T_endZ

213. Cartesian-TimeDiff-TZ-T_endX

214. Cartesian-TimeDiff-TZ-T_endY

215. Cartesian-TimeDiff-TZ-T_endZ

216. Cartesian-TimeDiff-T_endX-T_endY

217. Cartesian-TimeDiff-T_endX-T_endZ

218. Cartesian-TimeDiff-T_endY-T_endZ

219. areaQuotient_RT_3D

220. QRS-LoopArea

221. T-LoopArea

222. XQRS-1DIntegral

223. YQRS-1DIntegral

224. ZQRS-1DIntegral

225. XT-1DIntegral

226. YT-1DIntegral

227. ZT-1DIntegral

228. QuotientQRS_XY

229. QuotientQRS_XZ

230. QuotientQRS_YZ

231. QuotientT_XY

232. QuotientT_XZ

233. QuotientT_YZ

234. QRS-MagIntegral

235. T-MagIntegral

236. areaQuotient_RT_int

237. InterBeatTimeDiffX-QRS_beginX

238. InterBeatTimeDiffX-RX

239. InterBeatTimeDiffX-QRS_endX

240. InterBeatTimeDiffX-T_beginX

241. InterBeatTimeDiffX-TX

242. InterBeatTimeDiffX-T_endX

243. InterBeatTimeDiffY-QRS_beginY

244. InterBeatTimeDiffY-RY

245. InterBeatTimeDiffY-QRS_endY

246. InterBeatTimeDiffY-T_beginY

247. InterBeatTimeDiffY-TY

248. InterBeatTimeDiffY-T_endY

249. InterBeatTimeDiffZ-QRS_beginZ

250. InterBeatTimeDiffZ-RZ

251. InterBeatTimeDiffZ-QRS_endZ

252. InterBeatTimeDiffZ-T_beginZ

253. InterBeatTimeDiffZ-TX

254. InterBeatTimeDiffZ-T_endZ

255. Mag-Val-QRS_begin

256. Mag-Val-R

257. Mag-Val-QRS_end

258. Mag-Val-T_begin

# Fig. 11c

259. Mag-Val-T

260. Mag-Val-T_end

261. Azi-Val-QRS_begin

262. Azi-Val-R

263. Azi-Val-QRS_end

264. Azi-Val-T_begin

265. Azi-Val-T

266. Azi-Val-T_end

267. Ele-Val-QRS_begin

268. Ele-Val-R

269. Ele-Val-QRS_end

270. Ele-Val-T_begin

271. Ele-Val-T

272. Ele-Val-T_end

273. azi_RR_norm

274. azi_no_norm

275. ele_RR_norm

276. ele_no_norm

277. mag_RR_norm

278. mag_no_norm

279. muQRS

280. sigmaQRS

281. muT

282. sigmaQRS

283. superpositionT-QRS

284. sanzangle

285. age

286. weight

287. height

288. BMI

289. NaN_number

290. CrossCor_features(1)

291. CrossCor_features(2)

292. CrossCor_features(3)

# Fig. 11d

## Statistiken

1 Mittelwert

2 Varianz

3 Kurtosis

4 Schiefe

5 5% Quantil

6 95% Quantil

## Fig. 12

Fig. 13

Fig. 14

$$\begin{bmatrix} C_{11} & C_{12} & \cdots & C_{16} \\ C_{21} & C_{22} & \cdots & C_{26} \\ \vdots & & & \vdots \\ C_{61} & C_{62} & \cdots & C_{66} \end{bmatrix} \cdot \begin{bmatrix} \mu_x^{\,neu} \\ \mu_y^{\,neu} \\ \mu_z^{\,neu} \\ \sigma_x^{\,neu} \\ \sigma_y^{\,neu} \\ \sigma_z^{\,neu} \end{bmatrix} = \begin{bmatrix} PC1^{\,neu} \\ PC2^{\,neu} \\ PC3^{\,neu} \\ PC4^{\,neu} \\ PC5^{\,neu} \\ PC6^{\,neu} \end{bmatrix} \Big\} \, 0$$

$$\underbrace{\phantom{XXXXXXXXXXXX}}_{28} \qquad \underbrace{\phantom{XX}}_{26} \qquad \underbrace{\phantom{XX}}_{30}$$

## Fig. 15

## Fig. 16

**Fig. 17**

[r]

24

r (t₁)

r (t₂)

Fläche (R-Welle)

Fläche (T-Welle)

[t]

Fig. 18

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- EP 86429 B1
- WO 9936860 A
- WO 03057031 A1

**In der Beschreibung aufgeführte Nicht-Patentliteratur**

- R and T wave relationships in the lead I Electrocardiogram. **BLOOMFIELD D K**. JOURNAL OF ELECTROCARDIOLOGY. ELSEVIER SCIENCE, 01 April 1969, vol. 2, 167-170